# EUROPEAN PATENT SPECIFICATION

(11) **EP 1 542 739 B1**
(45) Date of publication and mention of the grant of the patent: **10.12.2008**
(21) Application number: 03759536.0
(22) Date of filing: 26.09.2003
(51) Int. Cl.: A61L 31/00, A61L 27/00, A61F 2/06, A61L 31/10

(54) **PERIVASCULAR WRAPS**
PERIVASKULÄRE HÜLLEN
ENVELOPPES PERIVASCULAIRES

(30) Priority: 26.09.2002 US 414714 P; 27.09.2002 US 414693 P
(43) Date of publication of application: 22.06.2005
(73) Proprietor: ANGIOTECH INTERNATIONAL AG, 6300 Zug (CH)
(72) Inventor: GRAVETT, David, M., Vancouver, British Columbia V5Z 1Y8 (CA); TOLEIKIS, Philip, M., Vancouver, British Columbia V6P 5N8 (CA); GUAN, Dechi, Vancouver, British Columbia V6P 3Y1 (CA); SIGNORE, Pierre, E., Vancouver, British Columbia V6K 1X9 (CA); SPENCER, Thomas, S., Bellingham, WA 98226 (US); WANG, Kaiyue, Vancouver, British Columbia V5T 1Y5 (CA)
(74) Representative: Gowshall, Jonathan Vallance
(86) International application number: PCT/US2003/030280
(87) International publication number: WO 2004/028583

(56) References cited:
- EP-A- 0 689 807
- EP-A- 1 155 689
- WO-A-00/40278
- WO-A-02/056790
- US-A1- 2003 003 094
- US-A1- 2004 013 703

## Description

### TECHNICAL FIELD

The present invention relates generally to compositions and methods for improving and maintaining the integrity of body passageways or cavities following surgery or injury, and more specifically, to compositions that include therapeutic agents which may be delivered to body passageways or cavities for the purpose of preventing and/or reducing a proliferative biological response that may obstruct or hinder the optimal functioning of the passageway or cavity.

### BACKGROUND OF THE INVENTION

Each year, thousands of people lose the ability to deliver sufficient blood to various limbs of the body. When blood vessels do fail, natural or artificial grafts may be used to restore vessel function. For example, patients who must undergo chronic injections or puncturing of their blood vessels may ultimately have the insulted blood vessel(s) die (*e.g.*, patient's suffering from end-stage renal failure require hemodialysis and multiple injections or punctures). Many artificial grafts, such as expanded polytetrafluoroethylene (ePTFE) or Dacron® (polyethylene terephthalate), have been designed to act, and have been used, as a replacement blood conduit. Hence, needles or other medical devices may be repeatedly used on an on-going basis to penetrate a graft without causing the death of a blood vessel.

Although these grafts have been used successfully for many years, many fail for a variety of reasons. For example, thrombus formation may arise from reduced blood flow due to intimal hyperplasia, which occurs at the venous anastomosis (*i.e.*, at the blood vessel-graft attachment site). The thrombus arising from intimal hyperplasia may result in graft occlusion and graft failure. Factors thought to contribute to the occurrence of intimal hyperplasia include, for example, changes in blood flow hemodynamics along with damage to the vessel endothelium, compliance differences between the graft and the blood vessel, and changes in blood vessel stress. The development of intimal hyperplasia arising from an arterio-venous bypass graft placement is only one of many examples whereby intimal hyperplasia may occur following device placement.

To increase the patency of these devices, a method of reducing the degree of intimal hyperplasia is required. In this regard, several systemic pharmacotherapies have been tried. For example, pharmacotherapeutic regimes have included systemic anti-platelet therapies, such as aspirin and heparin. While these treatments have demonstrated some degree of efficacy in reducing intimal hyperplasia in animal models, no efficacy has been demonstrated in clinical studies. Methods of local drug delivery to the inside of the vessel have also failed to produce efficacy in the clinic.

There exists a need in the art for improved compositions and methods for improving or maintaining the integrity of body passageways or cavities. The present invention addresses the problem associated with the existing procedures, offers significant advantages over existing procedures, and provides other related advantages.

### SUMMARY OF THE INVENTION

The present invention relates to perivascular wraps and methods for producing perivascular wraps as defined in the claims. The present disclosure relates generally to compositions and methods for improving or maintaining the integrity of body passageways or cavities following surgery or injury, and more specifically, to either polymer devices or compositions that include therapeutic agents (either with or without a carrier) which may be delivered to the external walls of body passageways or cavities for the purpose of preventing and/or reducing a proliferative biological response that may obstruct or hinder the optimal functioning of the passageway or cavity.

In one aspect, the instant invention as defined in the claims provides delivery devices that include a one or more therapeutic agents and a mesh, wherein the mesh includes a biodegradable polymer. The therapeutic agents may be utilized to treat or prevent a wide variety of conditions, including, for example, iatrogenic complications of arterial and venous catheterization, ePTFE graft placement, aortic dissection, cardiac rupture, aneurysm, cardiac valve dehiscence, passageway rupture and surgical wound repair. Another condition includes intimal hyperplasia, which may arise at various graft sites. For example, intimal hyperplasia may arise at an anastomotic site, such as at a venous anastomosis, an arterial anastomosis, an arteriovenous fistula, an arterial bypass, or an arteriovenous graft. Representative body passageways and cavities that may be treated include, for example, arteries, veins, the heart, the esophagus, the stomach, the duodenum, the small intestine, the large intestine, the biliary duct, the ureter, the bladder, the urethra, the lacrimal ducts, the trachea, bronchi, bronchiole, nasal passages (including the sinuses) and other airways, eustachian tubes, the external auditory and, the vas deferens and other passageways of the male reproductive tract, the uterus and fallopian tubes and the ventricular system (cerebrospinal fluid) of the brain and the spinal cord. Representative examples of cavities include, for example, the abdominal cavity, the buccal cavity, the peritoneal cavity, the pericardial cavity, the pelvic cavity, perivisceral cavity, pleural cavity, inguinal canal and uterine cavity.

In another aspect of the disclosure, a method for improving or maintaining a body passageway lumen or cavity integrity is described. The method includes delivering to an external portion of the body passageway or cavity a delivery device. The device includes a therapeutic agent and a mesh, wherein the mesh includes a biodegradable polymer. The method may be used, for example, for treatment or prevention iatrogenic complications of arterial and venous catheterization, complications of vascular dissection, complications of gastrointestinal passageway rupture and dissection, and restonotic complications associated with vascular surgery.

In yet another aspect of the disclosure, a method for treating or preventing intimal hyperplasia is described. The method includes delivering to an anastomotic site a delivery device. The device includes a therapeutic agent and a mesh, wherein the mesh includes a biodegradable polymer. Examples of anastomotic sites include a venous anastomosis, an arterial anastomosis, such as an arterial bypass, an arteriovenous fistula, and an arteriovenous graft. In one aspect, the device is delivered to an external portion of the anastomotic site.

In yet another aspect of the disclosure, a method for drug delivery is described. The method includes contacting an external portion of a body passageway or cavity with a delivery device. The device includes a therapeutic agent and a mesh, wherein the mesh includes a biodegradable polymer. Examples of conditions that may be treated or prevented with the described method include iatrogenic complications of arterial and venous catheterization, complications of vascular dissection, complications of gastrointestinal passageway rupture and dissection, restonotic complications associated with vascular surgery, and intimal hyperplasia.

In one aspect of the invention, delivery devices, compositions, and methods are provided that include a therapeutic agent and a mesh, wherein the mesh includes a biodegradable polymer. The mesh may be in the form of a woven, knit, or non-woven mesh. The therapeutic agents may be an integral part of the biodegradable polymer mesh (i.e., may reside within the fibers of the mesh) or may be coated on the mesh by painting, spraying, or dipping. The coated therapeutic agents may be in the form of a surface-adherent coating, mask, film, gel, foam, or mold. In one embodiment, the mesh is a woven mesh that has a weft that includes a first polymer and a warp that includes a second polymer. The degradation profile of the weft polymer may be different than or the same as the degradation profile of the warp polymer. In another embodiment, the device includes at least two layers of mesh. In one aspect, at least two of the at least two layers of mesh are fused together. The multilayer device may further include a film layer. The film layer may reside between two of the at least two layers of mesh. In yet another embodiment, a delivery device is described that includes a mesh, wherein the mesh includes a biodegradable polymer and a first therapeutic agent. The device may further include a film that includes a second therapeutic agent, which may have the same or a different composition than the first therapeutic agent.

In one aspect, the mesh includes a biodegradable polymer that is formed from one or more monomers selected from the group consisting of lactide, glycolide, e-caprolactone, trimethylene carbonate, 1,4-dioxan-2-one, 1,5-dioxepan-2-one, 1,4-dioxepan-2-one, hydroxyvalerate, and hydroxybutyrate. In one aspect, the polymer includes a copolymer of a lactide and a glycolide. In another aspect, the polymer includes a poly(caprolactone). In yet another aspect, the polymer includes a poly(lactic acid). In yet another aspect, the polymer includes a copolymer of lactide and e-caprolactone. In yet another aspect, the polymer includes a polyester (e.g., a poly(lactide-co-glycolide). The poly(lactide-coglycolide) may have a lactide:glycolide ratio ranges from about 20:80 to about 2:98, a lactide:glycolide ratio of about 10:90, or a lactide:glycolide ratio of about 5:95. In one aspect, the poly(lactide-co-glycolide) is poly(L-lactide-co-glycolide).

A wide variety of therapeutic agents may be utilized within the scope of the present invention, including for example microtubule stabilizing agents, anti-proliferative agents including cytotoxic and cytostatic agents, anti-angiogenic agents, and the like (*e.g.*, paclitaxel, or analogues or derivatives thereof), and other cell cycle inhibitors that may reduce the rate of cell proliferation. Furthermore, therapeutic drugs may include, but are not limited to, those agents that inhibit some or all of the processes involved in cell proliferation, cell migration, inflammation, and matrix deposition, such as in the development of intimal hyperplasia. In addition, therapeutic drugs may include, but are not limited to those agents that inhibit some or all of the processes involved in inflammation such as those involved in the development of intimal hyperplasia. In one aspect, the described devices include a therapeutic agent that is capable of inhibiting smooth muscle cell migration, proliferation, matrix production, inflammation, or a combination thereof. Agents included in one or more of these categories are anti-angiogenic agents, *e.g.*, anthracyclines (e.g., doxorubicin), fucoidon, and taxanes, and analogues or derivatives thereof; certain immunosuppressive compounds such as sirolimus (rapamycin), and analogues or derivatives thereof; certain anti-inflammatory agents, such as dexamethasone and analogues or derivatives thereof; certain antibiotic agents, e.g., dactinomycin and analogues or derivatives thereof; certain statins, such as cervistatin and analogues or derivatives thereof; and certain estrogens, e.g. 17-β-estradiol and analogues and derivatives thereof. Also included are those agents that have antithrombotic and/or antiplatelet properties such as clopidogrel, glycoprotein inhibitors (abciximab, eptifibatide, tirofiban and analogues and derivatives thereof. Each of these therapeutic agents may be used individually or in any combination thereof, and wherein some combinations results in synergistic effects. The delivery devices of the invention may be loaded with between about 0.001 mg/cm² to 5 mg/cm² of the therapeutic agent.

In one aspect, the device includes an anti-angiogenic agent, such as paclitaxel, fucoidon, doxorubicin, or an analogue or derivative thereof Delivery devices may be loaded with between about 0.001 mg/cm² to 5 mg/cm² of paclitaxel, or an analogue, or derivative thereof. In another aspect, the therapeutic agent includes an anti-inflammatory agent, such as dexamethasone or a statin (e.g., cervistatin or an analogue or derivative thereof). In another aspect, the therapeutic agent includes an antibiotic neoplastic agent, such as actinomycin or an analogue or derivative thereof. In yet another aspect, the therapeutic agent includes an estrogen, such as 17-β-estradiol or an analogue or derivative thereof. In yet another aspect, the therapeutic agent is an antibacterial agent, an antifungal agent, or an antiviral agent. In yet another aspect, the therapeutic agent is an immunosuppressive antibiotic, such as sirolimus (or an analogue or derivative thereof), everolimus, or tacrolimus.

The therapeutic agents of the disclosure may further include a polymeric or non-polymeric carrier. In one embodiment, the device may include a film that includes the polymeric carrier and the therapeutic agent. In other embodiments, the polymer carrier and the therapeutic agent may be formed into a wrap, gel, foam, mold, or a coating. Examples of carriers include, for example, poly(glycolic acid), poly(lactic acid), copolymers of lactic acid and glycolic acid, poly(caprolactone), copolymers of lactic acid and ε-caprolactone, poly(lactide), poly(glycolide), lactide-glycolide copolymers, lactide-caprolactone copolymers, block copolymers of an alkyloxide and hydroxy acid(s), block copolymers of an alkylene oxide and lactide, block copolymers of an alkylene oxide and lactide/glycolide, block copolymer of ethylene oxide and hydroxy acids, polyesters, poly(hydroxyl acids), poly(lactide-coglycolide), gelatin, hyaluronic acid, collagen matrices and albumin, as well as blends and combinations thereof. In other embodiments, the carrier is a poly(lactide-co-glycolide) having a lactide:glycolide ratio that ranges from about 100:0 to about 2:98, and other embodiments have a ratio of about 50:50. In yet another embodiment, the carrier is a block copolymer, wherein a first block includes methoxypolyethylene glycol and a second block includes a polyester, for example methoxy poly(ethylene glycol)-block-poly(D,L-lactide).

In one aspect, the polymeric carrier is biodegradable. In one aspect, the biodegradable polymeric carrier is formed from one or more monomers selected from the group consisting of lactide, glycolide, ε-caprolactone, trimethylene carbonate, 1,4-dioxan-2-one, 1,5-dioxepan-2-one, 1,4-dioxepan-2-one, hydroxyvalerate, or hydroxybutyrate. In another aspect, the biodegradable polymer carrier includes a copolymer of lactic acid and glycolic acid. In yet another aspect, the biodegradable polymer carrier includes a copolymer of lactide and glycolide. In yet another aspect, the biodegradable polymer carrier includes a copolymer of D,L-lactide and glycolide. In yet another aspect, the biodegradable polymer carrier includes poly(caprolactone). In yet another aspect, the biodegradable polymer carrier includes poly(lactic acid). In yet another aspect, the biodegradable polymer carrier includes a copolymer of lactide and ε-caprolactone. In yet another aspect, the biodegradable polymer carrier includes a block copolymer having a first block and a second block, wherein the first block includes methoxypolyethylene glycol and the second block includes a polyester. The polyester may include a polymer selected from the group consisting of a poly(lactide), a poly(glycolide), a poly(caprolactone), or a trimethylene carbonate polymer, poly(hydroxyl acid), poly(L-lactide) poly(D,L lactide), poly(D,L-lactide-co-glycolide), poly(L-lactide-co-glycolide), copolymers of lactic acid and glycolic acid, copolymers of ε-caprolactone and lactide, copolymers of glycolide and ε-caprolactone, copolymers of lactide and 1,4-dioxane-2-one, polymers and copolymers that includes one or more of the residue units of the monomers D-lactide, L-lactide, D,L-lactide, glycolide, ε-caprolactone, trimethylene carbonate, 1,4-dioxane-2-one or 1,5-dioxepan-2-one, and combinations and blends thereof. In one aspect, the poly(lactide) is poly(D,L-lactide). In another aspect, the polyester is formed from one or more monomers selected from the group consisting of lactide, glycolide, e-caprolactone, trimethylene carbonate, 1,4-dioxan-2-one, 1,5-dioxepan-2-one, 1,4-dioxepan-2-one, hydroxyvalerate, and hydroxybutyrate. The block copolymer may have a methoxypoly(ethylene glycol) : polyester ratio in the range of about 10:90 to about 30:70. In another aspect, the block copolymer has a methoxypoly(ethylene glycol) : polyester ratio of about 20:80. In one aspect, the methoxypoly(ethylene glycol) has a molecular weight range of about 200 g/mol to about 5000 g/mol. In another aspect, the molecular weight is about 750 g/mol.

In one embodiment, the biodegradable polymer carrier includes a block copolymer having an A-B-A structure. The A block includes polyoxyalkane, and the B block includes a polyester. In one aspect, the polyoxyalkane may be a polyethylene glycol, a poly(ethylene oxide-co-propylene oxide), and a poly(ethylene oxide-co-propylene oxide-co-ethylene oxide). In one aspect, the polyester may be a poly(lactide), a poly(glycolide), a poly(caprolactone), or a trimethylene carbonate polymer, poly(hydroxyl acids), poly(L-lactide) poly(D,L lactide), poly(D,L-lactide-co-glycolide), poly(L-lactide-co-glycolide), copolymers of lactic acid and glycolic acid, copolymers of ε-caprolactone and lactide, copolymers of glycolide and ε-caprolactone, copolymers of lactide and 1,4-dioxane-2-one, polymers and copolymers that include one or more of the residue units of the monomers D-lactide, L-lactide, D,L-lactide, glycolide, ε-caprolactone, trimethylene carbonate, 1,4-diaxane-2-one or 1,5-dioxepan-2-one, and combinations and blends thereof. In another aspect, the polyester is formed from one or more monomers selected from the group consisting of lactide, glycolide, ε-caprolactone, trimethylene carbonate, 1,4-dioxan-2-one, 1,5-dioxepan-2-one, 1,4-dioxepan-2-one, hydroxyvalerate, and hydroxybutyrate.

In another embodiment, the biodegradable polymer carrier includes a block copolymer having a B-A-B structure. The A block includes polyoxyalkane and the B block includes a polyester. The polyoxyalkane may be a polyethylene glycol, a poly(ethylene oxide-co-propylene oxide), and a poly(ethylene oxide-co-propylene oxide-co-ethylene oxide). In one aspect, the polyester may be a poly(lactide), a poly(glycolide), a poly(caprolactone), or a trimethylene carbonate polymer, poly(hydroxyl acids), poly(L-lactide) poly(D,L lactide), poly(D,L-lactide-co-glycolide), poly(L-lactide-co-glycolide), copolymers of lactic acid and glycolic acid, copolymers of ε-caprolactone and lactide, copolymers of glycolide and ε-caprolactone, copolymers of lactide and 1,4-dioxane-2-oue, polymers and copolymers that includes one or more of the residue units of the monomers D-lactide, L-lactide, D,L-lactide, glycolide, ε-caprolactone, trimethylene carbonate, 1,4-dioxane-2-one or 1,5-dioxepan-2-one, and combinations and blends thereof. In another aspect, the polyester is formed from one or more monomers selected from the group consisting of lactide, glycolide, ε-caprolactone, trimethylene carbonate, 1,4-dioxan-2-one, 1,5-dioxepan-2-one, 1,4-dioxepan-2-one, hydroxyvalerate, and hydroxybutyrate.

In another embodiment of the disclosure, the biodegradable polymer carrier may include hyaluronic acid, chitosan, or sodium algirate.

In yet another embodiment of the disclosure, the polymer carrier may include poly(urethane) or poly(hydroxyethylmethacrylate).

In another aspect of the disclosure, the carrier is a non-polymeric carrier. The non-polymeric carrier may have a viscosity of between about 100 and about 3x10⁶ centipoise or a melting point of greater than 10°C. Examples of non-polymeric carriers includes sucrose acetate isobutyrate, calcium stearate, a sucrose ester (*e.g.*, sucrose oleate). In certain embodiments of the disclosure, the carrier can be a wax, such as refined paraffin wax or microcrystalline wax.

In yet another aspect of the invention, a method of producing a delivery device is described. The method includes contracting component that include one or more therapeutic agents (optionally, in a polymeric carrier of the invention or non-polymeric carrier of the disclosure) and a biodegradable polymer, under conditions and for a time sufficient for the components to form a solid, and forming the solid into a delivery device. In one aspect, a biodegradable polymer in a viscous or a liquid form may be formed into solid fibers (*e.g.*, by extrusion). The fibers then may be weaved or knitted into a delivery device, which may, be formed into a wrap.

In yet another embodiment, a method of producing a delivery device is described that includes coating a mesh with one or more therapeutic agents, wherein the mesh includes a biodegradable polymer. The mesh may be coated by painting, dipping, or spraying. The coating may be in the form of a film or may include a gel or foam. Delivery devices produced by this method also may be formed into a wrap.

In yet another aspect, a composition is described that includes a therapeutic agent and a mesh, wherein the mesh includes a biodegradable polymer. Examples of therapeutic agents for use in the described compositions include paclitaxel, rapamycin, actinomycin, 17-β-estradiol, or an analogue or derivative thereof. The composition may include a statin (*e.g.*, lovastatin, simvastatin, pravastatin, fluvastatin, atorvastatin, cervistatin, and derivatives and analogues thereof). In yet another aspect, the therapeutic agent may be an anthracycline (*e.g.*, doxorubicin, daunorubicin, idarubicin, epirubicin, pirarubicin, zorubicin, carubicin, and derivatives, analogues, and combinations thereof) or an anti-inflammatory agent, such as, *e.g.*, corticosteroids, NTHEs, anti-inflammatory cytokines, and derivatives, analogues, and combinations thereof.

In yet another aspect, a delivery device is described that includes a mesh, wherein the mesh includes a copolymer of a lactide and glycolide, a therapeutic agent (paclitaxel or a derivative or analogue thereof), and a polymer carrier (methoxy poly(ethylene glycol)-block-poly(D,L-lactide)). In one aspect, the delivery device may be a perivascular wrap. The device may include between about 0.001 mg/cm² to 5 mg/cm² of the paclitaxel or derivative or analogue thereof.

In one particularly preferred embodiment of the disclosure, the delivery device including a therapeutic agent and a mesh, wherein the mesh includes a biodegradable polymer, is delivered to an artery or vein by direct application to an external site or to the adventitia. In addition to the uses described above, the compositions of this invention may have many different uses.

These and other aspects of the present invention will become evident upon reference to the following detailed description and attached drawings.

### BRIEF DESCRIPTION OF THE DRAWINGS

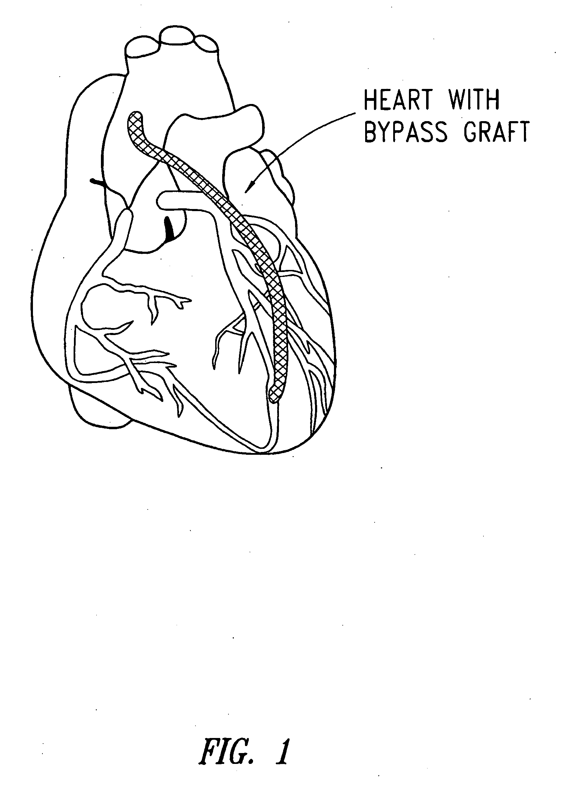

Figure 1 is a cartoon that shows a heart with a bypass graft.

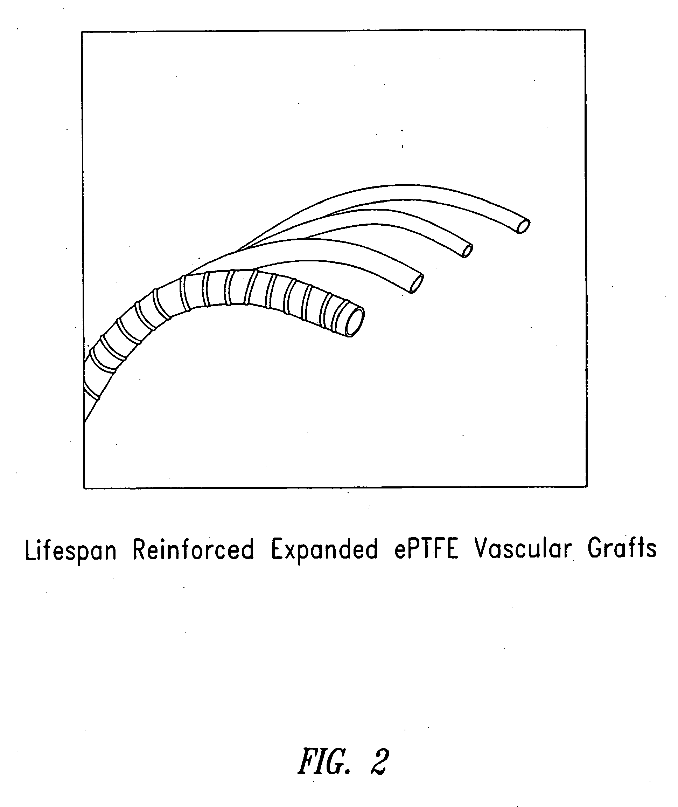

Figure 2 is a picture that shows expanded polytetrafluoroethylene (ePTFE) vascular grafts.
Figure 3 is a picture that shows an uninjured carotid artery from a rat balloon injury model.
Figure 4 is a picture that shows an injured carotid artery from a rat balloon injury model.
Figure 5 is a picture that shows a paclitaxel/mesh treated carotid artery in a rat balloon injury model (345 µg paclitaxel in a 50:50 PLG coating on a 10:90 PLG mesh).
Figure 6 is a cartoon that shows a schematic drawing of an artery-to-artery graft and showing the placement of the mesh wrap (not to scale).
Figure 7 is a cartoon that shows a schematic drawing of sectioning plan.
Figure 8 is a graph that shows the effect of paclitaxel, at different doses, on maximal intimal thickness.
Figure 9 is a graph that shows the effect of paclitaxel, at different doses, on intimal area.
Figure 10 is a graph that shows the effect of paclitaxel, at different doses, on percent stenosis.

### DETAILED DESCRIPTION OF THE INVENTION

Prior to setting forth the invention, it may be helpful to an understanding thereof to set forth definitions of certain terms that will be used hereinafter.

"Body Passageway" as used herein refers to any of number of passageways, tubes, pipes, tracts, canals, sinuses or conduits which have an inner lumen and allow the flow of materials within the body. Representative examples of body passageways include arteries and veins, lacrimal ducts, the trachea, bronchi, bronchiole, nasal passages (including the sinuses) and other airways, eustachian tubes, the external auditory canal, oral cavities, the esophagus, the stomach, the duodenum, the small intestine, the large intestine, biliary tracts, the ureter, the bladder, the urethra, the fallopian tubes, uterus, vagina and other passageways of the female reproductive tract, the vas deferens and other passageways of the male reproductive tract, and the ventricular system (cerebrospinal fluid) of the brain and the spinal cord.

"Body cavity" as used herein refers to any of number of hollow spaces within the body. Representative examples of cavities include, for example, the abdominal cavity, the buccal cavity, the peritoneal cavity, the pericardial cavity, the pelvic cavity, perivisceral cavity, pleural cavity, inguinal canal and uterine cavity.

"Therapeutic agent" as used herein refers to those agents which may mitigate, treat, cure or prevent (*e.g.*, as a prophylactic agent) a given disease or condition. Representative examples of therapeutic agents are discussed in more detail below, and include, for example, microtubule stabilizing agents, anti-angiogenic agents, cell cycle inhibitors, antithrombotic agents, antiplatelet agents, anti-inflammatory agents as well as cytokines and other factors involved in the wound healing or proliferation cascade. Briefly, within the context of the present invention, anti-angiogenic agents should be understood to include any protein, peptide, chemical, or other molecule, which acts to inhibit vascular growth (*see*, *e.g.*, U.S. Patent Nos. 5,994,341, 5,886,026, and 5,716,981).

Any concentration or other numerical ranges recited herein are to be understood to include concentrations of any integer within the range and fractions thereof, such as one tenth and one hundredth of an integer, unless otherwise indicated. It should be understood that the terms "a" and "an" as used above and elsewhere herein refer to "one or more" of the enumerated components. As used herein, the term "about" means ± 15% of an indicated value.

As noted above, the present disclosure relates generally to delivery devices, compositions, and methods for improving the integrity of body passageways following surgery or injury, that includes delivering to an external portion of the body passageway (*i.e.*, a non-luminal surface), a composition that includes a therapeutic agent, and within preferred embodiments, either a polymer alone or a composition including a therapeutic agent (with or without a polymeric carrier). Briefly, delivery of a therapeutic agent to an external portion of a body passageway (*e.g.*, quadrantically or circumferentially) avoids many of the disadvantages of traditional approaches. In addition, delivery of a therapeutic agent as described herein allows the administration of greater quantities of the therapeutic agent with less constraint upon the volume to be delivered. For example, in embodiments in which the therapeutic agent has been incorporated into or coated onto a mesh material, the device may deliver a therapeutically effective amount of the drug in a low total volume of material, thereby reducing the amount of polymer that is released into the body upon degradation.

In one aspect, the devices and compositions of the present invention are sterile. Many pharmaceuticals are manufactured to be sterile and this criterion is defined by the USP XXII <1211>. Sterilization in this embodiment may be accomplished by a number of means accepted in the industry and listed in the USP XXII <1211>, including gas sterilization, ionizing radiation, thermal treatments or filtration. Sterilization may be maintained by what is termed asceptic processing, defined also in USP XXII <1211>. Acceptable gases used for gas sterilization include ethylene oxide. Acceptable radiation types used for ionizing radiation methods include gamma, for instance from a cobalt 60 source, and electron beam. A typical dose of gamma radiation is 2.5x10⁴5v (2.5 MRad). When appropriate, filtration may be accomplished using a filter with suitable pore size, for example 0.22 µm and of a suitable material, for instance Teflon.

The therapeutic agents, therapeutic devices or compositions and pharmaceutical devices or compositions provided herein may be placed within one or more containers, along with packaging material that provide instructions regarding the use of such materials. These containers may or may not contain a dessicant. Generally, such instructions include a tangible expression describing the reagent concentration, as well as within certain embodiments, relative amounts of excipient ingredients or diluents (*e.g.*, water, saline or PBS) that may be necessary to reconstitute the pharmaceutical composition. The containers and contents therein may also be sterile.

Within yet another aspect of the disclosure, pharmaceutical devices, products, or compositions are provided, that includes (a) a therapeutic agent and a biodegradable polymer, wherein at least some of the biodegradable polymer is in the form of a mesh, in a container, and (b) a notice associated with the container in form prescribed by a governmental agency regulating the manufacture, use, or sale of devices or pharmaceuticals, which notice is reflective of approval by the agency of a device or compound that; for example, disrupts microtubule function or is anti-angiogenic or is anti-proliferative or is immunosuppressive and the like, for human or veterinary administration to treat non-tumorigenic angiogenesis-dependent diseases such as, for example, inflammatory arthritis or neovascular diseases of the eye. Briefly, Federal Law requires that the use of a pharmaceutical agent in the therapy of humans be approved by an agency of the Federal government. Responsibility for enforcement (in the United States) is with the Food and Drug Administration, which issues appropriate regulations for securing such approval, detailed in 21 U.S.C. §§ 301-392. Regulation for biological materials that include products made from the tissues of animals, is also provided under 42 U.S.C. § 262. Similar approval is required by most countries, although, regulations may vary from country to country.

A wide variety of therapeutic agents may be delivered to external portions of body passageways or cavities, either with or without a carrier (*e.g.*, polymeric or non-polymeric), in order to treat or prevent a condition associated with the body passageway or cavity. Discussed in more detail below are: I) Therapeutic Agents, II) Device Compositions, and III) Treatment or Prevention of Compromised Body Passageway or Cavity.

### I. THERAPEUTIC AGENTS

A wide variety of agents (also referred to herein as 'therapeutic agents' or 'drugs') may be utilized within the context of the present invention, either with or without a carrier (*e.g.*, a polymer; see section II below). Therapeutic drugs may include but are not limited to those agents which inhibit some or all of the processes involved in the development of intimal hyperplasia, such as cell proliferation, cell migration and matrix deposition. Agents in this category include cell cycle inhibitors and/or anti-angiogenic agents, *e.g.*, anthracyclines, fucoidon, and taxanes, certain immunosuppressive compounds such as sirolimus and analogues, and derivatives, certain nonsteroidal anti-inflammatory agents such as dexamethasone and analogues and derivatives, certain antibiotic agents such as dactinomycin and analogues, and derivatives, certain statins such as cervistatin and analogues and derivatives, and certain estrogens such as 17-β-estradiol and analogues and derivatives. Furthermore, antithrombotic agents and antiplatelet agents may be used. Discussed in more detail below are (A) anthracyclines (*e.g.*, doxorubicin and mitoxantrone), (B) taxanes (*e.g.*, paclitaxel and docetaxol), (C) sirolimus analogues. (D) antibiotic agents (*e.g.*, dactinomycin), (E) statins (*e.g.*, cervistatin), and (F) estrogens (*e.g.*, 17-β-estradiol).

### A. Anthracyclines

Anthracyclines have the following general structure, where the R groups may be a variety of organic groups:

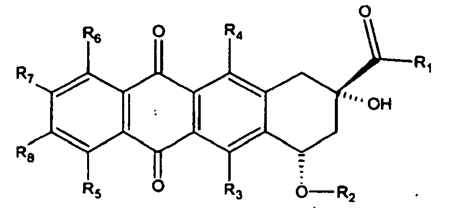

According to U.S. Patent 5,594,158, suitable R groups are as follows: R₁ is CH₃ or CH₂OH R₂ is daunosamine or H; R₃ and R₄ are independently one of OH, NO₂, NH₂, F, Cl, Br, I, CN, H or groups derived from these; R₅ is hydrogen, hydroxy, or methoxy; and R₆₋₈ are all hydrogen. Alternatively, R₅ and R₆ are hydrogen and R₇ and R₈ are alkyl or halogen, or vice versa.

According to U.S. Patent 5,843,903, R₁ may be a conjugated peptide. According to U.S. Patent 4,296,105, R₅ may be an ether linked alkyl group. According to U.S. Patent 4,215,062, R₅ may be OH or an ether linked alkyl group. R₁ may also be linked to the anthracycline ring by a group other than C(O), such as an alkyl or branched alkyl group having the C(O) linking moiety at its end, such as -CH₂CH(CH₂-X)C(O)-R₁, wherein X is H or an alkyl group (see, *e.g.*, U.S. Patent 4,215,062). R₂ may alternately be a group linked by the functional group =N-NHC(O)-Y, where Y is a group such as a phenyl or substituted phenyl ring. Alternately R₃ may have the following structure: 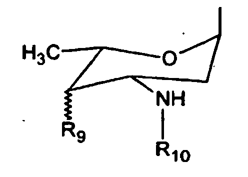 in which R₉ is OH either in or out of the plane of the ring, or is a second sugar moiety such as R₃. R₁₀ may be H or form a secondary amine with a group such as an aromatic group, saturated or partially saturated 5 or 6 membered heterocyclic having at least one ring nitrogen (*see* U.S. Patent 5,843,903). Alternately, R₁₀ may be derived from an amino acid, having the structure -C(O)CH(NHR₁₁)(R₁₂), in which R₁₁ is H, or forms a C₃₋₄ membered alkylene with R₁₂. R₁₂ may be H, alkyl, aminoalkyl, amino, hydroxy, mercapto, phenyl, benzyl or methylthio (*see* U.S. Patent 4,296,105).

Exemplary anthracyclines are Doxorubicin, Daunorubicin, Idarubicin, Epirubicin, Pirarubicin, Zorubicin, and Carubicin. Suitable compounds have the structures:

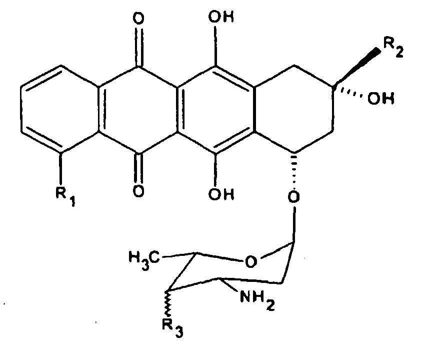

| | R₁ | R₂ | R₃ |
|---|---|---|---|
| Doxorubicin: | OCH₃ | C(O)CH₂OH | OH out of ring plane |
| Epirubicin: (4' epimer of doxorubicin) | OCH₃ | C(O)CH₂OH | OH in ring plane |
| Daunorubicin: | OCH₃ | C(O)CH₃ | OH out of ring plane |
| Idarubicin: | H | C(O)CH₃ | OH out of ring plane |
| Pirarubicin: | OCH₃ | C(O)CH₂OH | 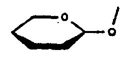 |
| Zorubicin: | OCH₃ | C(CH₃)(=N)NHC(O)C₆H₅ | OH |
| Carubicin: | OH | C(O)CH₃ | OH out of ring plane |

Other suitable anthracyclines are Anthramycin, Mitoxantrone, Menogaril, Nogalamycin, Aclacinomycin A, Olivomycin A, Chromomycin A₃, and Plicamycin having the structures:

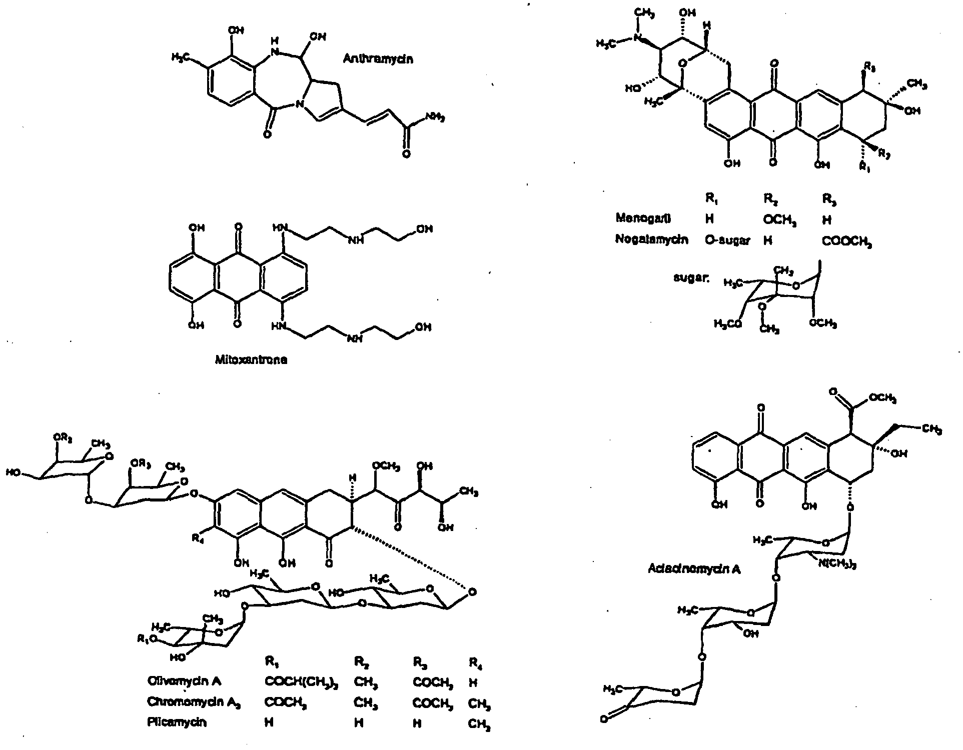

Other representative anthracyclines include, FCE 23762 doxorubicin derivative (Quaglia et al., J. Liq. Chromatogr. 17(18):3911-3923, 1994), annamycin (Zou et al., J. Pharm. Sci. 82(11):1151-1154, 1993), ruboxyl (Rapoport et al., J. Controlled Release 58(2):153-162, 1999), anthracycline disaccharide doxorubicin analogue (Pratesi et al., Clin. Cancer Res. 4(11):2833-2839, 1998), N-(trifluoroacetyl)doxorubicin and 4'-O-acetyl-N-(trifluoroacetyl)doxorubicin (Berube & Lepage, Synth. Commun. 28(6):1109-1116, 1998), 2-pyrrolinodoxorubicin (Nagy et al., Proc. Nat'l Acad. Sci. U.S.A. 95(4):1794-1799, 1998), disaccharide doxorubicin analogues (Arcamone et al., J. Nat'l Cancer Inst. 89(16):1217-1223, 1997), 4-demethoxy-7-O-[2,6-dideoxy-4-O-(2,3,6-trideoxy-3-amino-α-L-lyxo-hexopyranosyl)-α-L-lyxo-hexopyranosyl] adriamicinone doxorubicin disaccharide analog (Monteagudo et al., Carbohydr. Res. 300(1):11-16, 1997), 2-pyrrolinodoxorubicin (Nagy et al., Proc. Nat'l Acad. Sci. U.S.A. 94(2):652-656, 1997), morpholinyl doxorubicin analogues (Duran et al., Cancer Chemother. Pharmacol. 38(3):210-216, 1996), enaminomalonyl-β-alanine doxorubicin derivatives (Seitz et al., Tetrahedron Lett. 36(9):1413-16, 1995), cephalosporin doxorubicin derivatives (Vrudhula et al., J. Med. Chem. 38(8):1380-5, 1995), hydroxyrubicin (Solary et al., Int. J. Cancer 58(1):85-94, 1994), methoxymorpholino doxorubicin derivative (Kuhl et al., Cancer Chemother. Pharmacol. 33(1):10-16, 1993), (6-maleimidocaproyl)hydrazone doxorubicin derivative (Willner et al., Bioconjugate Chem. 4(6):521-7, 1993), N-(5,5-diacetoxypent-1-yl) doxorubicin (Cherif & Farquhar, J. Med. Chem. 35(17):3208-14, 1992), FCE 23762 methoxymorpholinyl doxorubicin derivative (Ripamonti et al., Br. J. Cancer 65(5):703-7, 1992), N-hydroxysuccinimide ester doxorubicin derivatives (Demant et al., Biochim. Biophys. Acta 1118(1):83-90, 1991), polydeoxynucleotide doxorubicin derivatives (Ruggiero et al., Biochim. Biophys. Acta 1129(3):294-302, 1991), morpholinyl doxorubicin derivatives (EPA 434960), mitoxantrone doxorubicin analogue (Krapcho et al., J. Med Chem. 34(8):2373-80. 1991), AD198 doxorubicin analogue (Traganos et al., Cancer Res. 51(14):3682-9, 1991), 4-demethoxy-3'-N-trifluoroacetyldoxorubicin (Horton et al., Drug Des. Delivery 6(2):123-9, 1990), 4'-epidoxorubicin (Drzewoski et al., Pol. J. Pharmacol. Pharm. 40(2):159-65, 1988; Weenen et al., Eur. J. Cancer Clin. Oncol. 20(7):919-26, 1984), alkylating cyanomorpholino doxorubicin derivative (Scudder et al., J. Nat'l Cancer Inst. 80(16):1294-8, 1988), deoxydihydroiodooxorubicin (EPA 275966), adriblastin (Kalishevskaya et al., Vestn. Mosk. Univ., 16(Biol. 1):21-7, 1988), 4'-deoxydoxorubicin (Schoelzel et al., Leuk Res. 10(12):1455-9, 1986), 4-demethyoxy-4'-o-methyldoxorubicin (Giuliani et al., Proc. Int. Congr. Chemother. 16:285-70-285-77, 1983), 3'-deamino-3'-hydroxydoxorubicin (Horton et al., J. Antibiot. 37(8):853-8, 1984), 4-demethyoxy doxorubicin analogues (Barbieri et al., Drugs Exp. Clin. Res. 10(2):85-90, 1984), N-L-leucyl doxorubicin derivatives (Trouet et al., Anthracyclines (Proc. Int. Symp. Tumor Pharmacother.), 179-81, 1983), 3'-deamino-3'-(4-methoxy-1-piperidinyl) doxorubicin derivatives (U.S. 4,314,054), 3'-deamino-3'-(4-mortholinyl) doxorubicin derivatives (U.S. 4,301,277), 4'-deoxydoxorubicin and 4'-o-methyldoxorubicin (Giuliani et al., Int. J. Cancer 27(1):5-13, 1981), aglycone doxorubicin derivatives (Chan & Watson, J. Pharm. Sci. 67(12):1748-52, 1978), SM 5887 (Pharma Japan 1468:20,1995), MX-2 (Pharma Japan 1420:19, 1994), 4'-deoxy-13(S)-dihydro-4'-iododoxorubicin (EP 275966), morpholinyl doxorubicin derivatives (EPA 434960), 3'-deamino-3'-(4-methoxy-1-piperidinyl) doxorubicin derivatives (U.S. 4,314,054), doxorubicin-14-valerate, morpholinodoxorubicin (U.S. 5,004,606), 3'-deamino-3'-(3"-cyano-4"-morpholinyl doxorubicin; 3'-deamino-3'-(3"-cyano-4"-morpholinyl)-13-dihydoxorubicin; (3'-deamino-3'-(3"-cyano-4"-morpholinyl) daunorubicin; 3'-deamino-3'-(3"-cyano-4"-morpholinyl)-3-dihydrodaunorubicin; and 3'-deamino-3'-(4"-morpholinyl-5-iminodoxorubicin and derivatives (U.S. 4,585,859), 3'-deamino-3'-(4-methoxy-1-piperidinyl) doxorubicin derivatives (U.S. 4,314,054) and 3-deamino-3-(4-morpholinyl) doxorubicin derivatives (U.S. 4,301,277).

### B. Taxanes

In another aspect, the therapeutic agent is a taxane, or a derivative or an analog thereof. Briefly, taxanes such as, for example, paclitaxel, are compounds that disrupt mitosis (M-phase) by binding to tubulin to form abnormal mitotic spindles.

The taxane paclitaxel is a highly derivatized diterpenoid (Wani et al., J. Am. Chem. Soc. 93:2325, 1971) which has been obtained from the harvested and dried bark of *Taxus brevifolia* (Pacific Yew) and *Taxomyces Andreanae* and *Endophytic Fungus* of the Pacific Yew (Stierle et al., Science 60:214-216, 1993). It has been formulated into commercial compositions, including the product TAXOL^{®}. Analogs and derivatives of paclitaxel include, for example, commercial products such as TAXOTERE^{®}, as well as compounds such as docetaxel, 10-desacetyl analogues of paclitaxel and 3'N-desbenzoyl-3'N-t-butoxy carbonyl analogues of paclitaxel) (*see generally* Schiff et al., Nature 277:665-667, 1979; Long and Fairchild, Cancer Research 54:4355-4361, 1994; Ringel and Horwitz, J. Nat'l Maycer Inst. 83(4):288-291, 1991; Pazdur et al., Cancer Treat. Rev. 19(4):351-386, 1993; WO 94/07882; WO 94/07881; WO 94/07880; WO 94/07876; WO 93/23555; WO 93/10076; WO94/00156; WO 93/24476; EP 590267; WO 94/20089; U.S. Patent Nos. 5,294,637; 5,283,253; 5,279,949; 5,274,137; 5,202,448; 5,200,534; 5,229,529; 5,254,580; 5,412,092; 5,395,850; 5,380,751; 5,350,866; 4,857,653; 5,272,171; 5,411,984; 5,248,796; 5,248,796; 5,422,364; 5,300,638; 5,294,637; 5,362,831; 5,440,056; 4,814,470; 5,278,324; 5,352,805; 5,411,984; 5,059,699; 4,942,184; Tetrahedron Letters 35(52):9709-9712, 1994; J. Med. Chem. 35:4230-4237, 1992; J. Med Chem. 34:992-998, 1991; J. Natural Prod. 57(10):1404-1410, 1994; J. Natural Prod 57(11):1580-1583, 1994; J. Am. Chem. Soc. 110:6558-6560, 1988). Taxanes may be made utilizing the techniques cited within the references provided herein, or, obtained from a variety of commercial sources, including for example, Sigma Chemical Co., St. Louis, Missouri (T7402 - from *Taxus brevifolia*).

Further representative examples of taxanes include 7-deoxy-docetaxol, 7,8-cyclopropataxanes, N-substituted 2-azetidones, 6,7-epoxy paclitaxels, 6,7-modified paclitaxels, 10-desacetoxytaxol, 10-deacetyltaxol (from 10-deacetylbaccatin III), phosphonooxy and carbonate derivatives of taxol, taxol 2',7-di(sodium 1,2-benzenedicarboxylate, 10-desacetoxy-11,12-dihydrotaxol-10,12(18)-diene derivatives, 10-desacetoxytaxol, Protaxol (2'-and/or 7-O-ester derivatives ), (2'-and/or 7-O-carbonate derivatives), asymmetric synthesis of taxol side chain, fluoro taxols, 9-deoxotaxane, (13-acetyl-9-deoxobaccatine III, 9-deoxotaxol, 7-deoxy-9-deoxotaxol, 10-desacetoxy-7-deoxy-9-deoxotaxol, Derivatives containing hydrogen or acetyl group and a hydroxy and tert-butoxycarbonylamino, sulfonated 2'-acryloyltaxol and sulfonated 2'-O-acyl acid taxol derivatives, succinyltaxol, 2'-γ-aminobutyryltaxol formate, 2'-acetyl taxol, 7-acetyl taxol, 7-glycine carbamate taxol, 2'-OH-7-PEG(5000) carbamate taxol, 2'-benzoyl and 2',7-dibenzoyl taxol derivatives, other prodrugs (2'-acetyltaxol; 2',7-diacetyltaxol; 2'succinyltaxol; 2'-(beta-alanyl)-taxol); 2'gamma-aminobutyryltaxol formate; ethylene glycol derivatives of 2'-succinyltaxol; 2'-glutaryltaxol; 2'-(N,N-dimethylglycyl) taxol; 2'-(2-(N,N-dimethylamino)propionyl)taxol; 2'orthocarboxybenzoyl taxol; 2'aliphatic carboxylic acid derivatives of taxol, Prodrugs {2'(N,N-diethylaminopropionyl)taxol, 2'(N,N-dimethylglycyl)taxol, 7(N,N-dimethylglycyl)taxol, 2',7-di-(N,N-dimethylglycyl)taxol, 7(N,N-diethylaminopropionyl)taxol, 2',7-di(N,N-diethylaminopropionyl)taxol, 2'-(L-glycyl)taxol, 7-(L-glycyl)taxol, 2',7-di(L-glycyl)taxol, 2'-(L-alanyl)taxol, 7-(L-alanyl)taxol, 2',7-di(L-alanyl)taxol, 2'-(L-leucyl)taxol, 7-(L-leucyl)taxol, 2',7-di(L-leucyl)taxol, 2'-(L-isoleucyl)taxol, 7-(L-isoleucyl)taxol, 2',7-di(L-isoleucyl)taxol, 2'-(L-valyl)taxol, 7-(L-valyl)taxol, 2'7-di(L-valyl)taxol, 2'-(L-phenylalanyl)taxol, 7-(L-phenylalanyl)taxol, 2',7-di(L-phenylalanyl)taxol, 2'-(L-prolyl)taxol, 7-(L-prolyl)taxol, 2',7-di(L-prolyl)taxol, 2'-(L-lysyl)taxol, 7-(L-lysyl)taxol, 2',7-di(L-lysyl)taxol, 2'-(L-glutamyl)taxol, 7-(L-glutamyl)taxol, 2',7-di(L-glutamyl)taxol, 2'-(L-arginyl)taxol, 7-(L-arginyl)taxol, 2',7-di(L-arginyl)taxol}, Taxol analogs with modified phenylisoserine side chains, taxotere, (N-debenzoyl-N-tert-(butoxycaronyl)-10-deacetyltaxol, and taxanes (*e.g.*, baccatin III, cephalomannine, 10-deacetylbaccatin III, brevifoliol, yunantaxusin and taxusin); and other taxane analogues and derivatives, including 14-beta-hydroxy-10 deacetybaccatin III, debenzoyl-2-acyl paclitaxel derivatives, benzoate paclitaxel derivatives, phosphonooxy and carbonate paclitaxel derivatives, sulfonated 2'-acryloyltaxol; sulfonated 2'-O-acyl acid paclitaxel derivatives, 18-site-substituted paclitaxel derivatives, chlorinated paclitaxel analogues, C4 methoxy ether paclitaxel derivatives, sulfenamide taxane derivatives, brominated paclitaxel analogues, Girard taxane derivatives, nitrophenyl paclitaxel, 10-deacetylated substituted paclitaxel derivatives, 14-β-hydroxy-10 deacetylbaccatin III taxane derivatives, C7 taxane derivatives, C10 taxane derivatives, 2-debenzoyl-2-acyl taxane derivatives, 2-debenzoyl and -2-acyl paclitaxel derivatives, taxane and baccatin III analogs bearing new C2 and C4 functional groups, n-acyl paclitaxel analogues, 10-deacetylbaccatin III and 7-protected-10-deacetylbaccatin III derivatives from 10-deacetyl taxol A, 10-deacetyl taxol B, and 10-deacetyl taxol, benzoate derivatives of taxol, 2-aroyl-4-acyl paclitaxel analogues, orthro-ester paclitaxel analogues, 2-aroyl-4-acyl paclitaxel analogues and 1-deoxy paclitaxel and 1-deoxy paclitaxel analogues.

In one aspect, the taxane has the formula (C 1): 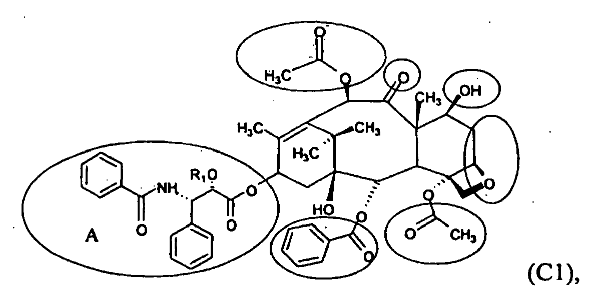 where the gray-highlighted portions may be substituted and the non-highlighted portion is the taxane core. A side-chain (labeled "A" in the diagram) is desirably present in order for the compound to have good activity. Examples of compounds having this structure include paclitaxel (Merck Index entry 7117), docetaxel (Taxotere, Merck Index entry 3458), and 3'-desphenyl-3'-(4-ntirophenyl)-N-debenzoyl-N-(t-butoxycarbonyl)-10-deacetyltaxol.

In one aspect, suitable taxanes such as paclitaxel and its analogs and derivatives are disclosed in Patent No. 5,440,056 as having the structure (C2): 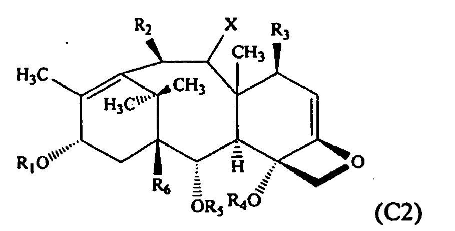 wherein X may be oxygen (paclitaxel), hydrogen (9-deoxy derivatives), thioacyl, or dihydroxyl precursors; R₁ is selected from paclitaxel or taxotere side chains or alkanoyl of the formula (C3) 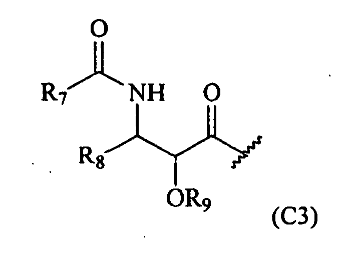 wherein R₇ is selected from hydrogen, alkyl, phenyl, alkoxy, amino, phenoxy (substituted or unsubstituted); R₈ is selected from hydrogen, alkyl, hydroxyalkyl, alkoxyalkyl, aminoalkyl, phenyl (substituted or unsubstituted), alpha or beta-naphthyl; and R₉ is selected from hydrogen, alkanoyl, substituted alkanoyl, and aminoalkanoyl; where substitutions refer to hydroxyl, sulfhydryl, allalkoxyl, carboxyl, halogen, thioalkoxyl, N,N-dimethylamino, alkylamino, dialkylamino, nitro, and -OSO₃H, and/or may refer to groups containing such substitutions; R₂ is selected from hydrogen or oxygen-containing groups, such as hydrogen, hydroxyl, alkoyl, alkanoyloxy, aminoalkanoyloxy, and peptidyalkanoyloxy; R₃ is selected from hydrogen or oxygen-containing groups, such as hydrogen, hydroxyl, alkoyl, alkanoyloxy, aminoalkanoyloxy, and peptidyalkanoyloxy, and may further be a silyl containing group or a sulphur containing group; R₄ is selected from acyl, alkyl, alkanoyl, aminoalkanoyl, peptidylalkanoyl and aroyl; R₅ is selected from acyl, alkyl, alkanoyl, aminoalkanoyl, peptidylalkanoyl and aroyl; R₆ is selected from hydrogen or oxygen-containing groups, such as hydrogen, hydroxyl alkoyl, alkanoyloxy, aminoalkanoyloxy, and peptidyalkanoyloxy.

In one aspect, the paclitaxel analogs and derivatives useful in the present invention are disclosed in PCT International Patent Application No. WO 93/10076. As disclosed in this publication, the analog or derivative should have a side chain attached to the taxane nucleus at C₁₃, as shown in the structure below (formula C4), in order to confer antitumor activity to the taxane.

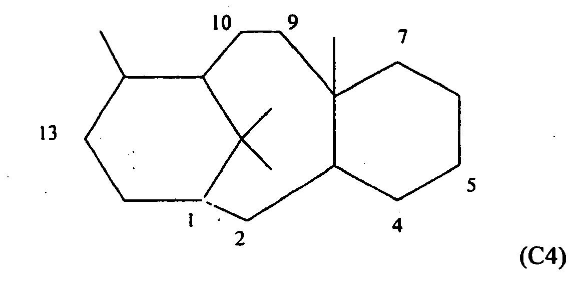

WO 93/10076 discloses that the taxane nucleus may be substituted at any position with the exception of the existing methyl groups. The substitutions may include, for example, hydrogen, alkanoyloxy, alkenoyloxy, aryloyloxy. In addition, oxo groups may be attached to carbons labeled 2, 4, 9, 10. As well, an oxetane ring may be attached at carbons 4 and 5. As well, an oxirane ring may be attached to the carbon labeled 4.

In one aspect, taxanes that are useful in the present invention are disclosed in U.S. Patent 5,440,056, which discloses 9-deoxo taxanes. These are compounds lacking an oxo group at the carbon labeled 9 in the taxane structure shown above (formula C4). The taxane ring may be substituted at the carbons labeled 1, 7 and 10 (independently) with H, OH, O-R, or O-CO-R where R is an alkyl or an aminoalkyl. As well, it may be substituted at carbons labeled 2 and 4 (independently) with aroyl, alkanoyl, aminoalkanoyl or alkyl groups. The side chain of formula (C3) may be substituted at R₇ and R₈ (independently) with phenyl rings, substituted phenyl rings, linear alkanes/alkenes, and groups containing H, O or N. R₉ may be substituted with H, or a substituted or unsubstituted alkanoyl group.

### C. Sirolimus

In another aspect, the therapeutic agent is sirolimus, or a derivative or an analog thereof. Briefly, sirolimus (also referred to as "rapamycin") is a macrolide antibiotic. Therapeutically the drug is classified as an immunosuppressant. Its mechanistic classification is as a cell cycle inhibitor and an mTORR (mammalian target of rapamycin) inhibitor. The structures of sirolimus, everolimus, and tacrolimus are provided below:

| Name | Code Name | Company | Structure | |
|---|---|---|---|---|
| Everolimus | SAR-943 | Novartis | See below | |
| Sirolimus Rapamune Rapamycin | AY-22989 NSC-226080 | Wyeth | See below | |
| Tacrolimus | FK506 | Fujusawa | See below | |
| Everolimus | 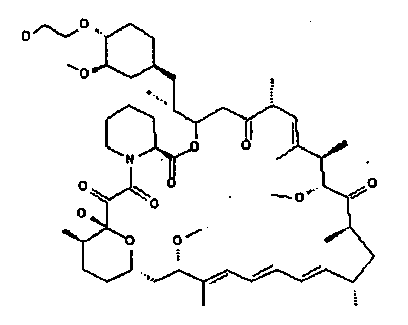 | | | |
| Tacrolimus | 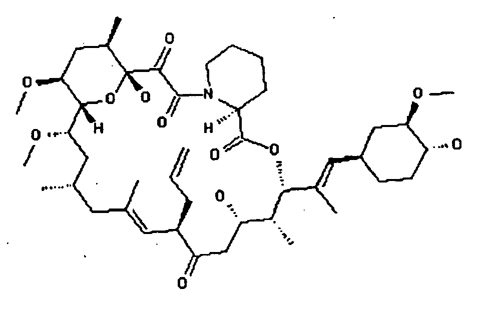 | | | |
| Sirolimus | 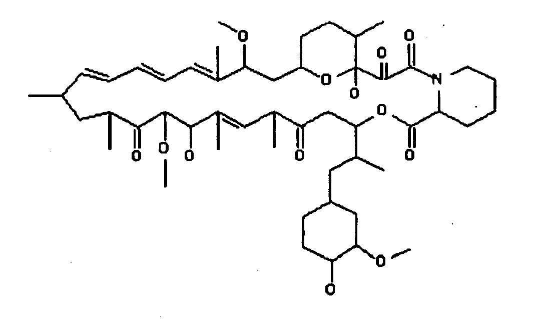 | | | |

Further sirolimus analogues and derivatives include tacrolimus and derivatives thereof (*e.g.*, EP0184162B1 and U.S. Patent No. 6,258,823) everolimus and derivatives thereof (*e.g.*, US Patent No. 5,665,772). Further representative examples of sirolimus analogues and derivatives include ABT-578 and others may be found in PCT Publication Nos. WO9710502, WO9641807, W09635423, W09603430, W09600282, WO9516691, WO9515328, WO9507468, W09504738, W09504060, W09425022, W09421644, WO9418207, WO9410843, W09409010, WO9404540, W09402485, WO9402137, W09402136, W09325533, WO9318043, WO9313663, W09311130, W09310122, W09304680, WO9214737, and WO9205179. Representative U.S. patents include U.S. Patent Nos. 6,342,507, 5,985,890, 5,604,234; 5,597,715, 5,583,139, 5,563,172, 5,561,228, 5,561,137, 5,541,193, 5,541,189, 5,534,632, 5,527,907, 5,484,799, 5,457,194, 5,457,182, 5,362,735, 5,324,644, 5,318,895, 5,310,903, 5,310,901, 5,258,389, 5,252,732, 5,247,076, 5,225,403, 5,221,625, 5,210,030, 5,208,241, 5,200,411, 5,198,421, 5,147,877, 5,140,018, 5,116,756, 5,109,112, 5,093,338, and 5,091,389.

### D. Anti-Inflammatory Agents

Another therapeutic agent useful in the instant invention includes anti-inflammatory agents. Anti-inflammatory agents include, without limitation, corticosteroids (*e.g.*, dexamethasone, hydrocortisone, triamcinolone), non-steroidal anti-inflammatory drugs (NTHEs) (*e.g.*, nabumetone, indomethicin, naproxen, ibuprofen), anti-inflammatory cytokines (*e.g.*, IL-4, IL-10, IL-13), cytokine antagonists (*e.g.*, IL-1 receptor antagonist, TNF-α monoclonal antibody, soluble TNF receptor, platelet factor 4), and the like. See also, *e.g.*, U.S. Patent No. 6,190,691; U.S. Patent No. 5,776,892; U.S. Patent No. 4,816,449; and U.S. Patent No. RE37,263.

### E. Actinomycin

In another aspect, the therapeutic agent is actinomycin, or a derivative or an analog thereof. Briefly, actinomycins are antibiotics isolated from a species of *Streptomyces*. Actinomycins are chromopeptides and most contain the chromophore, planar phenoxazone actinocin. Differences among actinomycins are confined to the peptide side chains which vary in the structure of the constituent amino acids. Therapeutically the drug is classified as an antibiotic neoplastic agent. Its mechanistic classification is as a cell cycle inhibitor.

### F. Statins

In another aspect, the therapeutic agent is a statin, or a derivative or an analog thereof. Briefly, statins are competitive inhibitors of 3-hydroxy-3-methylglutaryl coenzyme A reductase (HMG-CoA) which catalyses an early rate limiting step in cholesterol biosynthesis. Therapeutically the drugs are classified as therapeutics for dyslipidemia. The mechanistic classification is as HMG-CoA reductase inhibitors. These compounds may also have antiproliferative and antimigratory effects on cells. Representative statins include but are not limited to: lovastatin, simvastatin, pravastatin, fluvastatin, atorvastatin, and cervistatin.

### G. Estrogens

In another aspect, the therapeutic agent is an estrogen such as 17-*β*-estradiol, or a derivative or an analog thereof. Briefly, 17-*β*-estradiol is a steroidal estrogen. Therapeutically the drug is classified as an estrogen agonist. Additional effects include inhibition of cell migration and proliferation.

### II. DEVICE COMPOSITIONS

As noted above, therapeutic devices and compositions of the present disclosure comprise a biodegradable polymer and/or a non-degradable polymer, wherein at least some of the polymer is in the form of a mesh. The therapeutic devices and compositions of the present disclosure may additionally comprise a carrier, such as a polymeric or non-polymeric carrier. A wide variety of polymers and polymeric carriers of the invention may be utilized to contain and/or deliver one or more of the therapeutic agents discussed above, including for example both biodegradable and non-biodegradable compositions.

### MESH COMPOSITIONS

Representative examples of biodegradable compositions that may be used to prepare the mesh include polymers that comprise albumin, collagen, hyaluronic acid and derivatives, sodium alginate and derivatives, chitosan and derivatives gelatin, starch, cellulose polymers (for example methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextran and derivatives, polysaccharides, poly(caprolactone), fibrinogen, poly(hydroxyl acids), poly(L-lactide) poly(D,L lactide), poly(D,L-lactide-co-glycolide), poly(L-lactide-co-glycolide), copolymers of lactic acid and glycolic acid, copolymers of e-caprolactone and lactide, copolymers of glycolide and ε-caprolactone, copolymers of lactide and 1,4-dioxane-2-one, polymers and copolymers that include one or more of the residue units of the monomers D-lactide, L-lactide, D,L-lactide, glycolide, ε-caprolactone, trimethylene carbonate, 1,4-dioxane-2-one or 1,5-dioxepan-2-one, poly(glycolide), poly(hydroxybutyrate), poly(alkylcarbonate) and poly(orthoesters), polyesters, poly(hydroxyvaleric acid), polydioxanone, poly(ethylene terephthalate), poly(malic acid), poly(tartronic acid), polyanhydrides, polyphosphazenes, poly(amino acids). These compositions include copolymers of the above polymers as well as blends and combinations of the above polymers. (*see*, *generally*, Illum, L., Davids, S.S. (eds.) "Polymers in Controlled Drug Delivery" Wright, Bristol, 1987; Arshady, J. Controlled Release 17:1-22, 1991; Pitt, Int. J Phar. 59:173-196, 1990; Holland et al., J. Controlled Release 4:155-0180, 1986).

Representative examples of non-biodegradable polymers include ethylene-covinyl acetate copolymers, acrylic-based and methacrylic-based polymers [*e.g.*, poly(acrylic acid), poly(methylacrylic acid), poly(methylmethacrylate), poly(hydroxyethylmethacrylate), poly(alkylcynoacrylate), poly(alkyl acrylates), poly(alkyl methacrylates)], poly(ethylene), poly(proplene), polyamides [*e.g.*, nylon 6,6], poly(urethanes) [e.g. poly(ester urethanes), poly(ether urethanes), poly(carbonate urethanes), poly(ester-urea)], polyethers [poly(ethylene oxide), poly(propylene oxide), poly(ethylene oxide)-poly(propylene oxide) copolymers, diblock and triblock copolymers, poly(tetramethylene glycol)], silicone containing polymers and vinyl-based polymers [polyvinylpyrrolidone, poly(vinyl alcohol), poly(vinyl acetate phthalate), poly(styrene-co-isobutylene-co-styrene). These compositions include copolymers as well as blends, crosslinked compositions and combinations of the above polymers.

These compositions may also comprise a combination of the above-mentioned biodegradable and non-degradable polymers. Polymers that may also be used may be either anionic [*e.g.*, alginate, carrageenin, hyaluronic acid, dextran sulfate, chondroitin sulfate, carboxymethyl dextran, caboxymethyl cellulose and poly(acrylic acid)], or cationic [*e.g.*, chitosan, poly-l-lysine, polyethylenimine, and poly(allyl amine)] (*see generally*, Dunn et al., J. Applied Polymer Sci. 50:353, 1993; Cascone et al., J. Materials Sci.: Materials in Medicine 5:770, 1994; Shiraishi et al., Biol. Pharm. Bull. 16:1164, 1993; Thacharodi and Rao, Int'l J. Pharm. 120:115, 1995; Miyazaki et al., Int'l J. Pharm. 118:257, 1995). Particularly preferred polymers include poly(ethylene-co-vinyl acetate), poly(carbonate urethanes), poly(hydroxyl acids) [*e.g.*, poly(D,L-lactic acid) oligomers and polymers, poly(L-lactic acid) oligomers and polymers, poly(D-lactic acid) oligomers and polymers, poly(glycolic acid), copolymers of lactic acid and glycolic acid, copolymers of lactide and glycolide, poly(caprolactone), copolymers of lactide or glycolide and ε-caprolactone), poly(valerolactone), poly(anhydrides), copolymers prepared from caprolactone and/or lactide and/or glycolide and/or polyethylene glycol. These preferred compositions include combinations and bends of preferred polymers.

### CARRIER COMPOSITIONS

The polymeric carriers may include one or more biodegradable polymer(s), one or more non-degradable polymer(s) or a combination of one or more biodegradable polymer(s) and non-degradable polymer(s).

Representative examples of biodegradable compositions that may be used to prepare the carrier include albumin, collagen, hyaluronic acid and derivatives, sodium alginate and derivatives, chitosan and derivatives gelatin, starch, cellulose polymers (for example methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextran and derivatives, polysaccharides, poly(caprolactone), fibrinogen, poly(hydroxyl acids), poly(L-lactide) poly(D,L lactide), poly(D,L-lactide-co-glycolide), poly(L-lactide-co-glycolide), copolymers of lactic acid and glycolic acid, copolymers of ε-caprolactone and lactide, copolymers of glycolide and e-caprolactone, copolymers of lactide and 1,4-dioxane-2-one, polymers and copolymers that include one or more of the residue units of the monomers D-lactide, L-lactide, D,L-lactide, glycolide, ε-caprolactone, trimethylene carbonate, 1,4-dioxane-2-one or 1,5-dioxepan-2-one, poly(glycolide), poly(hydroxybutyrate), poly(alkylcarbonate) and poly(orthoesters), polyesters, poly(hydroxyvaleric acid), polydioxanone, poly(ethylene terephthalate), poly(malic acrid), poly(tartronic acid), polyanhydrides, polyphosphazenes, and poly(amino acids). These compositions include copolymers of the above polymers as well as blends and combinations of the above polymers.

Representative examples of non-biodegradable polymers include ethylene-covinyl acetate copolymers, acrylic-based and methacrylic-based polymers [*e.g.*, poly(acrylic acid), poly(methylacrylic acid), poly(methylmethacrylate), poly(hydroxyethylmethacrylate), poly(alkylcynoacrylate), poly(alkyl acrylates), poly(alkyl methacrylates)], poly(ethylene), poly(proplene), polyamides [*e.g.*, nylon 6,6], poly(urethanes) [e.g. poly(ester urethanes), poly(ether urethanes), poly(carbonate urethanes), poly(ester-urea)], polyethers [poly(ethylene oxide), poly(propylene oxide), poly(ethylene oxide)-poly(propylene oxide) copolymers, diblock and triblock copolymers, poly(tetramethylene glycol)], silicone containing polymers and vinyl-based polymers [polyvinylpyrrolidone, poly(vinyl alcohol), poly(vinyl acetate phthalate), and poly(styrene-co-isobutylene-co-styrene). These compositions include copolymers as well as blends, crosslinked compositions and combinations of the above polymers.

Preferred polymeric carriers are biodegradable, such as copolymers of lactic acid and glycolic acid, copolymers of lactide and glycolide, copolymers of lactic acid and ε-caprolactone), diblock copolymers (A-B) with block A that includes methoxypolyethylene glycol and block B that includes a polyester, for example methoxypoly(ethylene glycol) - co - poly(D,L-lactide), and triblock copolymers (A-B-A) or (B-A-B) with block A including polyoxyalkane and block B including a polyester. Preferred polyoxyalkane blocks include polyethylene glycol, poly(ethylene oxide-co-propylene oxide), and poly(ethylene oxide-co-propylene oxide-co-ethylene oxide). Other preferred polymeric carriers include poly(lactides), poly(glycolides), a poly(caprolactones), poly(L-lactide-co-glycolide), copolymers of lactic acid and glycolic acid, copolymers of ε-caprolactone and lactide, copolymers of glycolide and e-caprolactone, copolymers of lactide and 1,4-dioxane-2-one, polymers and copolymers including one or more of the residue units of the monomers D-lactide, L-lactide, D,L-lactide, glycolide, ε-caprolactone, trimethylene carbonate, 1,4-dioxane-2-one, 1,5-dioxepan-2-one, or trimethylene carbonates, and combinations and blends thereof. In yet other embodiments, preferred polymeric carriers are non-biodegradable, such as poly(urethanes) and poly(hydroxyethylmethacrylates).

In one embodiment of the disclosure, the therapeutic agent is incorporated in a non-polymeric carrier. Non-polymeric carriers may be biodegradable or non-biodegradable and may be combined with the biodegradable or non-biodegradable compositions described above. Non-polymeric carriers may be viscous (e.g., having a viscosity in the range of between about 100 and about 3x10⁶ centipoise) or may be solid (having a melting point greater than 10 °C) or a glass. Representative examples of non-polymeric carriers that may be used include sugar ester derivatives (*e.g.*, sucrose acetate isobutyrate, sucrose oleate, and the like), sugar amide derivatives, fatty acids, fatty acid salts (e.g. calcium stearate) lipids, waxes (e.g. refined paraffin wax, microcrystalline wax), and vitamins (*e.g.*, vitamin E)

### FORMULATION

Polymers and polymeric carriers may be fashioned in a variety of forms, such as a film, wrap, gel, foam, sheet, mold, mesh, coatings and the like. Preferred polymeric carriers may be formed into a film, wrap, gel, foam, sheet, mold, coating or a combination thereof. In other preferred embodiments, the polymer carrier and therapeutic agent are coated onto the delivery device (*e.g.*, polymeric mesh) for use in the methods described herein. In a preferred aspect, a delivery device, which is preferably in a viscous or solid form, is coated by a variety of methods, such as by painting, dipping, or spraying.

Polymers and polymeric carriers of the invention may also be fashioned to have particularly desired release characteristics and/or specific properties. For example, polymers and polymeric carriers may be fashioned to release a therapeutic agent upon exposure to a specific triggering event such as pH (*see, e.g.,* Heller et al., "Chemically Self-Regulated Drug Delivery Systems," in Polymers in Medicine III, Elsevier Science Publishers B.V., Amsterdam, 1988, pp. 175-188; Kang et al., J. Applied Polymer Sci. 48:343, 1993; Dong et al., J. Controlled Release 19:171, 1992; Dong and Hoffman, J. Controlled Release 15:141, 1991; Kim et al., J. Controlled Release 28:143, 1994; Cornejo-Bravo et al., J. Controlled Release 33:223, 1995; Wu and Lee, Pharm. Res. 10(10):1544-1547, 1993; Serres et al., Pharm. Res. 13:196, 1996; Peppas, "Fundamentals of pH- and Temperature-Sensitive Delivery Systems," in Gumy et al. (eds.), Pulsatile Drug Delivery, Wissenschaftliche Verlagsgesellschaft mbH, Stuttgart, 1993, pp. 41-55; Doelker, "Cellulose Derivatives," 1993, in Peppas and Langer (eds.), Biopolymers I, Springer-Verlag, Berlin, ), Kost et al., Advanced Drug Delivery Reviews, 46:125-148, 2001). Representative examples of pH-sensitive polymers include poly(acrylic acid) and its derivatives (including, for example, homopolymers such as poly(aminocarboxylic acid); poly(acrylic acid); poly(methyl acrylic acid)), copolymers of such homopolymers, and copolymers of poly(acrylic acid) and acrylmonomers such as those discussed above. Other pH sensitive polymers include polysaccharides such as cellulose acetate phthalate; hydroxypropylmethylcellulose phthalate; hydroxypropylmethylcellulose acetate succinate; cellulose acetate trimellilate; and chitosan. Yet other pH sensitive polymers include any mixture of a pH sensitive polymer and a watersoluble polymer. In a preferred embodiment, the device is a woven mesh having a weft including a first polymer and a warp including a second polymer, wherein the weft polymer has a degradation or release profile similar to the warp polymer. In another embodiment, the polymer or polymer carrier that includes the weft has a degradation or release profile that is shorter in duration than the polymer that includes the warp. In another embodiment, the polymer including the weft has a degradation or release profile that is longer in duration than the polymer including the warp.

Likewise, polymers and polymeric carriers may be fashioned to be temperature sensitive (*see, e.g.,* Sershen et al., Advanced Drug Delivery Reviews, 54:1225-1235, 2002; Chen et al., "Novel Hydrogels of a Temperature-Sensitive Pluronic Grafted to a Bioadhesive Polyacrylic Acid Backbone for Vaginal Drug Delivery," in Proceed. Intern. Symp. Control. Rel. Bioact. Mater. 22:167, Controlled Release Society, Inc., 1995; Okano, "Molecular Design of Stimuli-Responsive Hydrogels for Temporal Controlled Drug Delivery," in Proceed. Intern. Symp. Control. Rel. Bioact. Mater. 22:111, Controlled Release Society, Inc., 1995; Johnston et al., Pharm. Res. 9(3):425, 1992; Tung, Int'l J. Pharm. 107:85, 1994; Harsh and Gehrke, J. Controlled Release 17:175, 1991; Bae et al., Pharm. Res. 8(4):531, 1991; Dinarvand and D'Emanuele, J. Controlled Release 36:221, 1995; Yu and Grainger, "Novel Thermo-sensitive Amphiphilic Gels: Poly N-isopropylacrylamide-co-sodium acrylate-co-n-N-alkylacrylamide Network Synthesis and Physicochemical Characterization," Dept. of Chemical & Bioligal Sci., Oregon Graduate Institute of Science & Technology, Beaverton, OR, pp. 820-821; Zhou and Smid, "Physical Hydrogels of Associative Star Polymers," Polymer Research Institute, Dept. of Chemistry, College of Environmental Science and Forestry, State Univ. of New York, Syracuse, NY, pp. 822-823; Hoffman et al., "Characterizing Pore Sizes and Water 'Structure' in Stimuli-Responsive Hydrogels," Center for Bioengineering, Univ. of Washington, Seattle, WA, p. 828; Yu and Grainger, "Thermo-sensitive Swelling Behavior in Crosslinked N-isopropylacrylamide Networks: Cationic, Anionic and Ampholytic Hydrogels," Dept. of Chemical & Biological Sci., Oregon Graduate Institute of Science & Technology, Beaverton, OR, pp. 829-830; Kim et al., Pharm. Res. 9(3):283-290, 1992; Bae et al., Pharm. Res. 8(5):624-628, 1991; Kono et al., J. Controlled Release 30:69, 1994; Yoshida et al., J. Controlled Release 32:97, 1994; Okano et al., J. Controlled Release 36:125, 1995; Chun and Kim, J. Controlled Release 38:39-47, 1996; D'Emanuele and Dinarvand, Int'l J. Pharm. 118:237, 1995; Katono et al., J. Controlled Release 16:215, 1991; Hoffman, "Thermally Reversible Hydrogels Containing Biologically Active Species," in Migliaresi et al. (eds.), Polymers in Medicine III, Elsevier Science Publishers B.V., Amsterdam, 1988, pp. 161-167; Hoffman, "Applications of Thermally Reversible Polymers and Hydrogels in Therapeutics and Diagnostics," in Third International Symposium on Recent Advances in Drug Delivery Systems, Salt Lake City, UT, Feb. 24-27, 1987, pp. 297-305; Gutowska et al., J. Controlled Release 22:95-104, 1992; Palasis and Gehrke, J. Controlled Release 18:1-12, 1992; Paavola et al., Pharm. Res. 12(12):1997-2002, 1995).

Representative examples of thermogelling polymers include homopolymers such as poly(N-methyl-N-n-propylacrylamide), LCST=19.8°C; poly(N-n-propylacrylamide), 21.5; poly(N-methyl-N-isopropylacrylamide), 22.3; poly(N-n-propylmethacrylamide), 28.0; poly(N-isopropylacrylamide), 30.9; poly(N, n-diethylacrylamide), 32.0; poly(N-isopropylmethacrylamide), 44.0; poly(N-cyclopropylacrylamide), 45.5; poly(N-ethylmethyacrylamide), 50.0; poly(N-methyl-N-ethylacrylamide), 56.0; poly(N-cyclopropylmethacrylamide), 59.0; poly(N-ethylacrylamide), 72.0. Moreover thermogelling polymers may be made by preparing copolymers between (among) monomers of the above, or by combining such homopolymers with other water soluble polymers (*e.g.*, poly(acrylic acid), poly(methylacrylic acid), poly(acrylate), poly(butyl methacrylate), poly(acrylamide) and poly(N-n-butyl acrylamide) and derivatives thereof.

Other representative examples of thermogelling polymers include cellulose ether derivatives such as hydroxypropyl cellulose, 41°C; methyl cellulose, 55°C; hydroxypropylmethyl cellulose, 66°C; and ethylhydroxyethyl cellulose, copolymers of α-hydroxy acid and poly(ethylene glycol) and Pluronics™ such as F-127; L-122; L-92; L-81; and L-61.

A wide variety of forms may be fashioned by the polymer and carriers of the present invention, including for example, coatings, threads, braids, knitted or woven sheets, tubes and rod-shaped devices, (*see, e.g.,* Goodell et al., Am. J. Hosp. Pharm. 43:1454-1461, 1986; Langer et al., "Controlled release of macromolecules from polymers", in Biomedical polymers, Polymeric materials and pharmaceuticals for biomedical use, Goldberg, E.P., Nakagim, A. (eds.) Academic Press, pp. 113-137, 1980; Rhine et al., J. Pharm. Sci. 69:265-270, 1980; Brown et al., J. Pharm. Sci. 72:1181, 1983; and Bawa et al., J. Controlled Release 1:259, 1985). Therapeutic agents may be incorporated into the device by, for example, occlusion in the polymer or in the void volume of a mesh material, dissolution in the polymer matrix, coating onto, and by binding the agent(s) to the device via covalent or non-covalent linkages. The therapeutic agents may be incorporated into a secondary carrier (*e.g.*, microparticles, microspheres, nanospheres, micelles, liposomes and/or emulsions) that is then incorporated into the primary carrier as described above. Within certain preferred embodiments of the invention, therapeutic compositions are provided in formulations such as knitted or woven meshes, pastes, sheets, films, particulates, tubes, gels, foams, braids, and sprays.

Preferably, therapeutic devices or compositions of the present invention are fashioned in a manner appropriate to the intended use. For example, a therapeutic agent and biodegradable polymer are formed into a mesh or wrap for application to a venous or arterial anastomosis, preferably on the external portion of the anastomosis. Within certain aspects of the present invention, the therapeutic device or composition should be biocompatible, and release one or more therapeutic agents over a period of several days to months with the specific release profile being appropriate for the specific indication being treated. Further, therapeutic compositions of the present invention should preferably be stable for several months and capable of being produced and maintained under sterile conditions.

In one preferred embodiment of the disclosure, a delivery device is provided that includes a therapeutic agent and a biodegradable polymer, wherein at least some of the biodegradable polymer is in the form of a mesh. A mesh of the invention, as used herein, is a material composed of a plurality of fibers or filaments (i.e., a fibrous material), where the fibers or filaments are arranged in such a manner (e.g., interwoven, knotted, braided, overlapping, looped, knitted, interlaced, intertwined, webbed, felted, and the like) so as to form a porous structure. Typically, a mesh is a pliable material, such that it has sufficient flexibility to be wrapped around the external surface of a body passageway or cavity, or a portion thereof. The mesh is capable of providing support to the structure (e.g., the vessel or cavity wall) and may be adapted to release an amount of the therapeutic agent.

A mesh may include fibers or filaments that are randomly oriented relative to each other or that are arranged in an ordered array or pattern. In one embodiment, for example, a mesh may be in the form of a fabric, such as, for example, a knitted, braided, crocheted, woven, non-woven (e.g., a melt-blown or wet-laid) or webbed fabric. In one embodiment, a mesh may include a natural or synthetic biodegradable polymer that may be formed into a knit mesh, a weave mesh, a sprayed mesh, a web mesh, a braided mesh, a looped mesh, and the like. Preferably, a mesh or wrap has intertwined threads that form a porous structure, which may be, for example, knitted, woven, or webbed. Representative examples of meshes include surgical meshes, such as those commercially available from Ethicon, Inc. (Somerville, NJ) under the trade designation VICRYL knitted mesh, VICRYL woven mesh, Prolene mesh, Mersilene Mesh, and those available from CR Bard (Murray Hill, NJ) under the trade designation Bard® Visilex® Mesh, Bard® Dulex™ Mesh, and Bard® Mesh Flat Sheets.

The structure and properties of the mesh used in a device depend on the application and the desired mechanical (i.e., flexibility, tensile strength, and elasticity), degradation properties, and the desired loading and release characteristics for the selected therapeutic agent(s). The mesh should have mechanical properties, such that the device will remain sufficiently strong until the surrounding tissue has healed. Factors that affect the flexibility and mechanical strength of the mesh include, for example, the porosity, fabric thickness, fiber diameter, polymer composition (e.g., type of monomers and initiators), process conditions, and the additives that are used to prepare the material.

Typically, the delivery device includes a mesh that possesses sufficient porosity to permit the flow of fluids through the pores of the fiber network and to facilitate tissue ingrowth. Generally, the interstices of the mesh or wrap should be sufficiently wide apart to allow light visible by eye, or fluids, to pass through the pores. However, materials having a more compact structure also may be used. The flow of fluid through the interstices of the mesh depends on a variety of factors, including, for example, the stitch count or thread density. The porosity of the mesh may be further tailored by, for example, filling the interstices of the mesh with another material (e.g., particles or polymer) or by processing the mesh (e.g., by heating) in order to reduce the pore size and to create non-fibrous areas. Fluid flow through the mesh or wrap of the invention will vary depending on the properties of the fluid, such as viscosity, hydrophilicity/hydrophobicity, ionic concentration, temperature, elasticity, pseudoplasticity, particulate content, and the like. Preferably, the interstices do not prevent the release of impregnated or coated therapeutic agent(s) from the mesh, and the interstices preferably do not prevent the exchange of tissue fluid at the application site.

Mesh materials should be sufficiently flexible so as to be capable of being wrapped around all or a portion of the external surface of a body passageway or cavity. Flexible mesh materials are typically in the form of flexible woven or knitted sheets having a thickness ranging from about 25 microns to about 3000 microns; preferably from about 50 to about 1000 microns. Mesh material suitable for wrapping around arteries and veins typically ranges from about 100 to 400 microns in thickness.

The diameter and length of the fibers or filaments may range in size depending on the form of the material (e.g., knit, woven, or non-woven), and the desired elasticity, porosity, surface area, flexibility, and tensile strength. The fibers may be of any length, ranging from short filaments to long threads (i.e., several microns to hundreds of meters in length). Depending on the application, the fibers may have a monofilament or a multifilament construction.

The mesh may include fibers that are of same dimension or of different dimensions, and the fibers may be formed from the same or different types of biodegradable polymers. Woven materials, for example, may include a regular or irregular array of warp and weft strands and may include one type of polymer in the weft direction and another type (having the same or a different degradation profile from the first polymer) in the warp direction. Similarly, knit materials may include one or more types (e.g., monofilament, multifilament) and sizes of fibers and may include fibers made from the same or from different types of biodegradable polymers.

The structure of the mesh (*e.g.*, fiber density and porosity) may impact the amount of therapeutic agent that may be loaded into the device. For example, a fabric having a loose weave characterized by a low fiber density and high porosity will have a lower thread count, resulting in a reduced total fiber volume and surface area. As a result, the amount of agent that may be loaded into or onto, with a fixed carrier: therapeutic agent ratio, the fibers will be lower than for a fabric having a high fiber density and lower porosity. It is preferable that the mesh also should not invoke biologically detrimental inflammatory or toxic response, should be capable of being fully metabolized in the body, have an acceptable shelf life, and be easily sterilized.

The delivery device may include multiple mesh materials in any combination or arrangement. For example, a portion of the device may be a knitted material and another portion may be a woven material. In another embodiment, the device may more than one layer (*e.g.*, a layer of woven material fused to a layer of knitted material or to another layer of the same type or a different type of woven material). In some embodiments, multi-layer constructions (e.g., device having two or more layers of material) may be used, for example, to enhance the performance properties of the device (e.g. for enhancing the rigidity or for altering the porosity, elasticity, or tensile strength of the device) or for increasing the amount of drug loading.

Multi-layer constructions may be useful, for example, in devices containing more than one type of therapeutic agent. For example, a first layer of mesh material may be loaded with one type of agent and a second layer may be loaded with another type of agent. The two layers may be unconnected or connected (*e.g.*, fused together, such as by heart welding or ultrasonic welding) and may be formed of the same type of fabric or from a different type of fabric having a different polymer composition and/or structure.

Preferably, the device, including the therapeutic agent as an integral part of the device or coated on the device, has a form useful for application to an external or internal portion of a body passageway or cavity to treat or prevent a condition leading to reduced integrity of such passageways or cavities.

In certain aspects, a mesh may include portions that are not in the form of a mesh. For example, the device may include the form of a film, sheet, paste, and the like, and combinations thereof. Within yet other aspects of the invention, the therapeutic compositions of the present invention may be formed as a film. Preferably, such films are generally less than 5, 4, 3, 2, or 1, mm thick, more preferably less than 0.75 mm or 0.5 mm thick, and most preferably less than 500 µm to 20 µm thick. Such films are preferably flexible with an appropriate tensile strength. Within certain embodiments of the invention, the therapeutic compositions may also comprise additional ingredients such as surfactants (*e.g.*, Pluronics™ such as F-127, L-122, L-92, L-81, and L-61), anti-oxidants [*e.g.* vitamin E], and hydrating agents [e*.*g*.* maltose trehelose, poly(ethylene glycol)].

In one embodiment, the device includes a multi-layer construction having a film layer that includes the therapeutic agent and one or more layers of mesh material. For example, the film layer may be interposed between two layers of mesh or may be disposed on just one side the mesh material. The film layer may include a first therapeutic agent, whereas one or more of the layers of mesh may include the same or a different agent. For example, in one embodiment, a device suitable for wrapping around a vein or artery includes a layer of mesh and a film layer loaded with a therapeutic agent. The device may be wrapped around a body passageway or cavity, such that the film layer contacts the external surface of the passageway or cavity. Thus, the device may deliver the appropriate dosage of agent and may provide sufficient mechanical strength to improve and maintain the structural integrity of the body passageway or cavity.

In certain aspects, a mesh may include other components, such as other biological agents or non-biodegradable agents or polymers. Examples of additional components include antibiotic and antimicrobial agents, waxes, radio-opaque or echogenic materials and magnetic resonance imaging (MRI) responsive materials (i.e., MRI contrast agents) to enable visualization of the device under ultrasound, fluoroscopy and/or MRI: For example, a delivery device may be made with or coated with a composition which is echogenic or radiopaque (*e.g.*, made with echogenic or radiopaque with materials such as powdered tantalum, tungsten, barium carbonate, bismuth oxide, barium sulfate, or, by the addition of microspheres or bubbles which present an acoustic interface). For visualization under MRI, contrast agents (*e.g.*, Gadolinium (III) chelates or iron oxide compounds) may be incorporated into the device, such as, for example, as a component in a coating or within the void volume of the device (*e.g.*, within a lumen, reservoir, or within the structural material used to form the device).

As noted above, the polymer and carrier compositions of the present invention may be formulated in a variety of forms to produce a delivery device suitable for application to the outside surface of a body passageway or cavity. Further, the compositions of the present invention may be formulated to contain one or more therapeutic agent(s), to contain a variety of additional compounds. and/or to have certain physical properties (*e.g.*, elasticity, a particular melting point or a specified release rate). Within certain embodiments of the invention, compositions may be combined in order to achieve a desired effect (*e.g.*, several preparations of microspheres may be combined in order to achieve both a quick and a slow or prolonged release of one or more factors).

The compositions of the present invention may be administered in combination with other therapeutic agents, pharmaceutically or physiologically acceptable carrier, excipients or diluents.

In one embodiment, the composition of the invention is in the form of a knitted or woven mesh. One or more therapeutic agents may be incorporated into the mesh using several different methods. In one embodiment, the therapeutic agent may be incorporated directly in the polymeric material that is used to produce the mesh. For example, the therapeutic agent may be admixed into a melt-processable composition that includes the biodegradable polymer. Using standard melt-processing techniques, fiber including a therapeutic agent may be prepared. These fibers may be used to prepare the desired mesh. In another embodiment, the therapeutic agent may be coated directly onto or absorbed into the polymeric thread/yarn that is used to prepare the mesh. In another embodiment, the therapeutic agent may be incorporated into a carrier composition that is then coated onto the polymeric thread/yarn that is used to produce the mesh. In another embodiment, the therapeutic agent may be coated onto or absorbed directly into the polymer that has already been knitted or woven into a mesh form. In another embodiment, the therapeutic agent may be incorporated into a carrier composition that is then coated onto the polymer that has already been knitted or woven into a mesh form. The therapeutic agent or the therapeutic agent/carrier composition may be applied using the various coating methods that are known in the art (*e.g.*, dip coating, spray coating, solvent casting, extrusion, roll coating, etc.). In some embodiments, the therapeutic agent may be attached directly to the fibers (*e.g.*, by physisorption, chemisorption, ligand/receptor interaction, covalent bonds, hydrogen bonds, ionic bonds, and the like). The fibers (either before or after incorporation into the mesh), optionally, may be pre-treated prior to application of the therapeutic agent to enhance adhesion and/or to introduce reactive sites for attaching the drug or an intermediate (*e.g.*, a linker) to the material. Surface treatment techniques are well known in the art and include, for example, applying a priming solution, plasma treatment, corona treatment, radiation treatment and surface hydrolysis, oxidation or reduction.

The instant disclosure also provides methods of making the devices and compositions including a therapeutic agent and a biodegradable polymer, wherein at least some of the biodegradable polymer is in the form of a mesh. In one embodiment, there is provided a method of producing a delivery device, including (a) contacting a therapeutic agent and a biodegradable polymer, under conditions and for a time sufficient for the therapeutic agent and biodegradable polymer to form a solid, and (b) weaving or knitting the solid into a delivery device. The biodegradable polymer of step (a) may be in a viscous form or a liquid form. In another embodiment of the diclosure, a preferred method of producing a delivery device, includes (a) contacting a biodegradable polymer and a therapeutic agent, wherein at least some of the biodegradable polymer is in the form of a mesh, and (b) placing the biodegradable polymer mesh and therapeutic agent under conditions and for a time sufficient for the mesh to form a solid delivery device. In yet another preferred embodiment of the disclosure, a delivery device may be produced by coating a biodegradable polymer with a therapeutic agent, wherein at least some of the biodegradable polymer is in the form of a mesh. Preferably the polymer mesh of the invention is coated by painting, dipping, or spraying, and the coat is in the form of a surface adherent coating, film, wrap, gel, foam, and the like.

In one embodiment, the polymer used to prepare the knitted or woven mesh includes a biodegradable polymer, as discussed herein. The preferred biodegradable polymer is one that may be spun into a yam that may then be knitted or woven into a mesh using the various techniques known in the art. Fibers having dimensions appropriate for preparing knit and woven fabrics may be made using standard melt-processing techniques, such as injection molding, compression molding, extrusion, electrospinning, melt spinning, solution spinning and gel state spinning. In other embodiments, the mesh is a random, non-woven network of fibers or filaments. Non-woven materials may be prepared, for example, by melt-blowing, wet-laying, or electrospinning the biodegradable polymer into the form of a fabric. Techniques for preparing biodegradable melt-blown fabrics are well known to those skilled in the art and are described, for example, in Wadsworth L., et al., "Melt Processing of PLA Resin into Nonwovens", 3rd Annual TANDEC Conference, Knoxville, 1993 and U.S. Patent No. 5,702,826.

The delivery device may provide controlled, sustained release of the therapeutic agent. Following implantation, the therapeutic agent is released from the biodegradable polymer as the polymer is degraded in the body. The rate of degradation depends on a variety of factors, such as the chemical composition, crystallinity, porosity, and wettability of the polymer. Examples of biodegradable polymers include biodegradable polyester and copolymers formed from lactide (e.g., L-Lactide) and glycolide. Preferably, poly(lactide-co-glycolide) polymers have a lactide / glycolide molar ratio between about 100/0 and about 2/98; preferably between about 15/85 and about 3/97; and most preferably between about 10/90 and about 3/97.

In one embodiment, the therapeutic agent may be incorporated into a carrier that is a polymer. The preferred polymeric carrier is a biodegradable polymer, such as a poly(ester) or a poly(ester)-poly(ether) copolymer. Preferred poly(ester) polymers are prepared from one or more hydroxy acids (*e.g.*, lactic acid, glycolic acid etc) or hydroxyl acid derivatives (*e.g.*, lactide, glycolide, caprolactone, etc.). The preferred hydroxyl acid derivatives are lactide and glycolide. The preferred carrier polymer has a lactide:glycolide molar ratio of about 85:15 to about 15:85. The more preferred carrier polymer has a lactide:glycolide molar ratio of about 85:15 to about 40:60.

The preferred poly(ester)-poly(ether) copolymer includes a diblock (A-B) or triblock (A-B-A, B-A-B) copolymer in which the block comprise either a poly(ester) or a poly(ether). U.S. Patent Nos. 5,612,052; 5,714,159; and 6,413,539 describe the preparation of poly(ester)-poly(ether) polymers. The preferred poly(ether) block includes a polyalkylene oxide. The preferred polyalkylene oxide includes poly(ethylene glycol) or poly(ethylene oxide). The preferred poly(ester) block is prepared from one or more hydroxy acids (*e.g.*, lactic acid, glycolic acid etc) or hydroxy acid derivatives (*e.g.*, lactide, glycolide, caprolactone etc). The preferred hydroxyl acid derivatives are lactide and glycolide. The preferred carrier polymer has a lactide:glycolide molar ratio of about 100:0 to about 15:85. The more preferred carrier polymer has a lactide:glycolide molar ratio of about 100:0 to about 40:60. The most preferred lactide:glycolide molar ratio is about 100:0. The preferred lactide isomer is D,L-lactide.

A preferred carrier diblock is an A-B.diblock copolymer wherein the A block includes methoxy poly(ethylene glycol) [MePEG] and the B block includes a poly(lactide). The methoxy poly(ethylene glycol) [MePEG] may have a molecular weight (Mn) in the range of about 200 g/mol to about 20,000 g/mol. The more preferred methoxy poly(ethylene glycol) may have a molecular weight (Mn) in the range of about 500 g/mol to about 2000 g/mol. The most preferred methoxy poly(ethylene glycol) may have a molecular weight (Mn) of about 750 g/mol. The poly(lactide) may have a molecular weight in the range of about 200 g/mol to about 10,000 g/mol. The more preferred molecular weight range for the poly(lactide) block is from about 500 g/mol to about 5000 g/mol.

A preferred carrier A-B diblock copolymer has a MePEG:lactide ratio (weight/weight) in the range of about 5:95 to about 40:60. The more preferred carrier A-B diblock copolymer has a MePEG:lactide ratio (weight/weight) in the range of about 10:90 to about 30:70. The most preferred carrier A-B diblock copolymer has a MePEG:lactide ratio (weight/weight) of about 20:80.

The therapeutic agent may be incorporated into the carrier using methods known in the art, such as addition of a solvent with subsequent removal of the solvent, dissolution of a therapeutic agent directly into the carrier and blending the therapeutic agent with the carrier. The methods used for incorporation of the therapeutic agent into the non-polymeric carrier are similar to those used to incorporate the therapeutic agent into the polymeric carrier, as described above.

The compositions may be sterile either by preparing them under an aseptic environment and/or they may be terminally sterilized using methods available in the art. A combination of both of these methods may also be used to prepare the composition in the sterile form. The most preferable method of sterilization is a terminal sterilization using gamma radiation or electron beam sterilization methods.

In one embodiment of the disclosure the composition may be packaged in a container. This container may comprise a polymer or a metal foil or a paper product or a combination of these. When the polymers used are polymers that degrade via hydrolysis, the composition may be packaged in a container that reduces the amount of water absorption by the product compared to the composition that is not packaged in such a container. In another embodiment of the disclosure, the container in which the composition is packaged may contain dessicant. In another embodiment of the disclosure the container packaged composition may be packaged in a secondary container that is more resistant to moisture permeation than the first or primary container of the composition. In another embodiment of the disclosure, a dessicant may be placed between the primary and secondary container. Properties of a container that may be important acceptable light transmission characteristics in order to prevent light energy from damaging the composition in the container (refer to USP XXII <661>), an acceptable limit of extractables within the container material (refer to USP XXII), an acceptable barrier capacity for moisture (refer to USP XXII <671>) or oxygen. In the case of oxygen penetration, this may be controlled by including in the container, a positive pressure of an inert gas, such as high purity nitrogen, or a noble gas, such as argon. The term "USP" refers to U.S. Pharmacopeia (*see* www.usp.org, Rockville, MD).

As discussed in more detail below, therapeutic agents of the present invention, which are optionally incorporated within one of the carriers described herein to form a therapeutic composition, may be prepared and utilized to treat or prevent a wide variety of conditions.

### III. TREATMENT OR PREVENTION OF COMPROMISED BODY PASSAGEWAY OR CAVITY

As noted above, the present disclosure relates generally to compositions and methods for improving the integrity of body passageways or cavities following surgery or injury, and more specifically, to compositions that include therapeutic agents which may be delivered to the external walls of body passageways or cavities for the purpose of preventing and/or reducing a proliferative biological response that may obstruct or hinder the optimal functioning of the passageway or cavity, including, for example, iatrogenic complications of arterial and venous catheterization, aortic dissection, cardiac rupture, aneurysm, cardiac valve dehiscence, graft placement (e.g. A-V-bypass, peripheral bypass, CABG), fistula formation, passageway rupture and surgical wound repair.

In certain embodiments of the disclosure, preferred methods for improving or maintaining a body passageway lumen or cavity includes delivering to an external portion of the body passageway or cavity a delivery device as described herein, for treating or preventing iatrogenic complications of arterial and venous catheterization, complications of vascular dissection, complications of gastrointestinal passageway rupture and dissection, complications associated with vascular surgery, and the like. Exemplary body passageways for use of the instant disclosure include arteries, veins, the heart, the esophagus, the stomach, the duodenum, the small intestine, the large intestine, biliary tracts, the ureter, the bladder, the urethra, lacrimal ducts, the trachea, bronchi, bronchiole, nasal airways, eustachian tubes, the external auditory canal, vas deferens and fallopian tubes. Exemplary cavities for use of the instant disclosure include the abdominal cavity, the buccal cavity, the peritoneal cavity, the pericardial cavity, the pelvic cavity, perivisceral cavity, pleural cavity and uterine cavity.

In order to further the understanding of such conditions, representative complications leading to a compromised body passageway or cavity integrity are discussed in more detail below.

### Cardiac Bypass Surgery

Coronary artery bypass graft ("CABG") surgery was introduced in the 1950s, and still remains a highly invasive, open surgical procedure, although less invasive surgical techniques are being developed. CABG surgery is a surgical procedure that is performed to overcome many types of coronary artery blockages. The purpose of bypass surgery is to increase the circulation and nourishment to the heart muscle that has been reduced due to arterial blockage. This procedure involves the surgeon accessing the heart and the diseased arteries, usually through an incision in the middle of the chest. Often, healthy arteries or veins are "harvested" from the patient to create "bypass grafts" that channel the needed blood flow around the blocked portions of the coronary arteries. The arteries or veins are connected from the aorta to the surface of the heart beyond the blockages thereby forming an autologous graft. This allows the blood to flow through these grafts and "bypass" the narrowed or closed vessel. The use of synthetic graft materials to create the "bypass" has been limited due to the lack of the appropriate biocompatibility of these synthetic grafts. CABG has significant short term limitations, including medical complications, such as stroke, multiple organ dysfunction, inflammatory response, respiratory failure and post-operative bleeding, each of which may result in death. Another problem associated with CABG is restenosis. Restenosis is typically defined as a renarrowing of an arterial blood vessel within six months of the CABG procedure. It typically occurs in approximately 25% to 45% of patients, and is the result of an excessive healing response to arterial injury after a revascularization procedure. Restenosis may occur within a short period following a procedure or may develop over the course of months or years. Longer term or " late" restenosis may result from excessive proliferation of scar tissue at the treatment site, the causes of which are not well understood. Thus any product that may reduce the incidence or magnitude of the restenotic process following CABG surgery would greatly enhance the well-being of a patient.

In order to prevent the restenotic complications associated with CABG surgery, such as those discussed above, a wide variety of therapeutic agents (with or without a carrier) /polymer compositions may be delivered to the external portion of the blood vessel. The polymer or therapeutic agent/polymer composition would be applied to the external portion of the vessel following the interventional or surgical procedure in order to prevent the restenotic complications.

Particularly preferred therapeutic agents either alone or in combination include microtubule stabilizing agents and other cell cycle inhibitors, anti-angiogenic agents, anti-inflammatory agents, immunosuppressive agents, antithrombotic agents, antiplatelet agents and other factors involved in the prevention or reduction of the restenotic process.

### Peripheral Bypass Surgery

Peripheral arterial disease (PAD) refers to diseases of any of the blood vessels outside of the heart. PAD is a range of disorders that may affect the blood vessels in the hands, arms, legs, or feet. The most common form of PAD is atherosclerosis. Atherosclerosis is a gradual process in which cholesterol and scar tissue build up in the arteries to form a substance called plaque. This build -up causes a gradual narrowing of the artery, which leads to a decrease in the amount of blood flow through that artery. When the flow of blood decreases, it results in a decrease of oxygen and nutrient supply to the body's tissues, which in turn may result in pain sensation. When the arteries to the legs are affected, the most common symptom is pain in the calf when walking. This is known as intermittent claudication.

Peripheral bypass surgery is a procedure to bypass an area of stenosed (narrowed) or blocked artery that is a result of atherosclerosis. In this surgical procedure, a synthetic graft (artificial blood vessels) or a-an autologous graft, vein, will be implanted to provide blood flow around the diseased area. First, the surgeon makes an incision in the leg, thigh, calf or ankle skin. The location of the incision may vary based on which vessels need to be bypassed and where there is healthy artery to connect to maintain the blood flow. The bypass graft is sewn into the artery above the stenosis or blockage, and below the stenosis or blockage. This bypass provides a means whereby blood will reach the tissue that has not been receiving enough blood and oxygen. Synthetic bypass grafts used in the legs are usually made of ePTFE.

Restenosis and occlusion of bypass grafts are one of the most important problems in peripheral bypass surgery. This restenosis is caused by neointimal growth (hyperplasia) and is especially pronounced within artificial graft material. This restenosis is usually at the anastomotic site where the graft and artery are connected via a surgical procedure. The intimal tissue typically grows from the native vessel into the graft. In order to prevent the restenotic complications associated with peripheral bypass surgery, such as those discussed above, a wide variety of therapeutic agents (with or without a carrier) /polymer compositions may be delivered to the external portion of the blood vessel. The polymer or therapeutic agent/polymer composition would be applied to the external portion of the vessel/anastomotic site following the interventional or surgical procedure in order to prevent the restenotic complications.

Particularly preferred therapeutic agents include microtubule stabilizing agents and other cell-cycle inhibitors, anti-angiogenic agents, anti-inflammatory agents, immunosuppressive agents, antithrombotic agents, antiplatelet agents and other factors in which may help the prevention or reduction of the restenotic process.

### Arterio-Venous (AV) Fistula

The arterio-venous (AV) fistula is surgically created vascular connection which allows the flow of blood from an artery directly to a vein. The AV fistula was first created by researchers for kidney failure patients who must undergo kidney dialysis.

Hemodialysis requires a viable artery and vein to draw blood from and return it to the body. The repeated puncturing often either causes a vein or artery to fail or causes other complications for the patient. The AV fistula increases the amount of possible puncture sites for hemodialysis and minimizes the damage to the patient's natural blood vessels. The connection that is created between the vein and artery forms a large blood vessel that continuously supplies an increased blood flow for performing hemodialysis.

Restenosis and eventual occlusion are one of the most important problems in the long term patency of the AV fistula. In order to prevent the restenotic complications associated with the surgical formation of an AV fistula, a wide variety of therapeutic agents (with or without a carrier) /polymer compositions may be delivered to the external portion of the blood vessel. The polymer or therapeutic agent/polymer composition would be applied to the external portion of the vessel/anastomotic site following the interventional or surgical procedure in order to prevent the restenotic complications.

Particularly preferred therapeutic agents include alone or in combination, microtubule stabilizing agents and other cell cycle inhibitors, anti-angiogenic agents, anti-inflammatory agents, immunosuppressive agents, antithrombotic agents, antiplatelet agents and other factors involved in the prevention or reduction of the restenotic process. The preferred composition is the therapeutic agent that is contained within a polymeric mesh.

### Arterio-Venous (AV) Graft Surgery

The AV graft surgical procedure is used for similar application as those for the AV fistula (e.g. hemodialysis patients). For the AV graft surgery, a synthetic graft material is used to connect the artery to the vein rather that the direct connection of the artery to the vein as is the case for the AV fistula. The incidence of intimal hyperplasia, which leads to occlusion of the graft, is one of the main factors that affect the long term patency of these grafts. This intimal hyperplasia may occur at the venous anastomosis and at the floor of the vein. A product that may reduce or prevent this occurrence of intimal hyperplasia will increase the duration of patency of these grafts. In order to reduce the occurrence of intimal hyperplasia at the venous anastomosis of an AV graft, a wide variety of therapeutic agents (with or without a carrier) /polymer compositions may be delivered to the external portion of the blood vessel. The polymer or therapeutic agent/polymer composition would be applied to the external portion of the vessel/anastomotic site following the interventional or surgical procedure in order to prevent the restenotic complications.

Particularly preferred therapeutic agents include alone or in combination, microtubule stabilizing agents and other cell cycle inhibitors, anti-angiogenic agents, anti-inflammatory agents, immunosuppressive agents, antithrombotic agents, antiplatelet agents and other factors involved in the prevention or reduction of the restenotic process. The preferred composition is the therapeutic agent that is contained within a polymeric mesh.

### Anastomotic Closure Devices

Anastomotic closure devices provide a means for rapidly repairing an anastomosis. The use of some of these devices requires an invasive surgical procedure. In one embodiment of this disclosure, following the use of an anastomotic closure device, the mesh containing the therapeutic agent may be wrapped around the anastomosis and the anastomotic closure device, if it is left at the surgical site.

In one embodiment, the diclosure provides a method for treating or preventing intimal hyperplasia that includes delivering to an anastomotic site a delivery device. The device includes a therapeutic agent and a biodegradable polymer, wherein at least some of the biodegradable polymer is in the form of a mesh. Exemplary anastomotic sites include venous anastomosis, arterial anastomosis, arteriovenous fistula, arterial bypass, and arteriovenous graft. Preferably, the device includes a polymer mesh with a therapeutic agent is delivered to an external portion of an anastomotic site.

### Transplant Applications

There are many applications in which various organs in the human body fail to function in a manner to sustain the well being of the patient. When an appropriate donor organ is available, an impaired organ may be replaced by a donor organ (*e.g.*, lung, heart, kidney etc). One of the potential complications following these transplant surgeries is the potential for stenosis to occur in the blood vessels at or near the anastomotic site between the donor and recipient vessels. For example, transplant renal artery stenosis is a complication that may occur following a kidney transplant. Transplant renal artery stenosis is when the artery from the abdominal aorta to the kidney narrows, limiting blood flow to the kidney. This may also make it difficult to keep blood pressure under control. Treatment typically involves expanding the narrowed segment using a small balloon.

One method to treat this stenosis is to apply the composition of this disclosure around the anastomotic site (junction of the donor and recipient vessels) in a perivascular manner. In a similar manner, the composition of this invention may be applied in a peritubular manner to the exterior surfaces of the trachea and or bronchi following a lung transplant procedure. Particularly preferred therapeutic agents include alone or in combination, microtubule stabilizing agents and other cell cycle inhibitors, anti-angiogenic agents, anti-inflammatory agents, immunosuppressive agents, anti-thrombotic agents, anti-platelet agents and other factors involved in the prevention or reduction of the stenotic process.

### Administration

As noted above, therapeutic agents, therapeutic compositions, or pharmaceutical compositions provided herein may be prepared for administration by a variety of different routes, including, for example, directly to a body passageway or cavity under direct vision (*e.g.*, at the time of surgery or via endoscopic procedures) or via percutaneous drug delivery to the exterior (adventitial) surface of the body passageway (*e.g.*, peritubular delivery). Other representative routes of administration include gastroscopy, ECRP and colonoscopy, which do not require full operating procedures and hospitalization, but may require the presence of medical personnel.

Briefly, peritubular drug delivery involves percutaneous administration of localized (often sustained release) therapeutic formulations using a needle or catheter directed *via* ultrasound, CT, fluoroscopic, MRI or endoscopic guidance to the disease site. Alternatively, the procedure may be performed intra-operatively under direct vision or with additional imaging guidance. Such a procedure may also be performed in conjunction with endovascular procedures, such as angioplasty, atherectomy or stenting or in association with an operative arterial procedure, such as endarterectomy, vessel or graft repair or graft insertion.

For example, in one embodiment of the disclosure, the mesh (with a therapeutic agent, such as paclitaxel) may be wrapped, either completely or partially, around an injured blood vessel (*e.g.*, following a surgical procedure, such as a graft insertion), a body tube (*e.g.*, trachea), and applied to the adventitial surface of a body passageway or cavity, which would allow drug concentrations to remain elevated for prolonged periods in regions where biological activity is most needed. For example paclitaxel may be delivered in a slow release form (*e.g.*, via a mesh) that contains from 0.001 mg/cm² to 5 mg/cm² (preferably 0.03 to 0.3 mg/cm²) over a selected period of time (*e.g.*, 1 to 120 days). For percutaneous administration, the agent may be administered at a dosage of 0.001 mg/ml to 30 mg/ml over a period of between 1 day and 90 days. In another embodiment of the disclosure, similar dose ranges may be used for Sirolimus and analogues and derivatives, dactinomycin and analogues and derivatives, cervistatin and analogues and derivatives, 17-β-estradiol and analogues and derivatives, dexamethasone and analogues and derivatives, and doxorubicin and analogues and derivatives as examples of compounds from the specific groupings described above. In yet another embodiment of the disclosure, the mesh (with a therapeutic agent, such as paclitaxel) may be placed in the appropriate location of a body cavity or a tumor resection site. If required, the mesh may be secured to the graft or the adjacent tissue using a surgical sealant, sutures, or surgical clips. For application at a tumor resection site, paclitaxel or other cell cycle inhibitor or microtubule stabilizing agent may be delivered in a slow release form (*e.g.*, via a mesh) that delivers from 0.01 mg/cm² to 20 mg/cm² mg/m² (preferably 0.01 to 10.0 mg/cm²) over a selected period of time (e.g., 1 to 150 days).

In another embodiment of the disclosure, the therapeutic agent may be delivered to an external portion of a body passageway or cavity, such as around an injured blood vessel (*e.g.*, following a surgical procedure, such as a graft insertion), a body tube (*e.g.*, trachea). For example, the therapeutic agent may be applied to the adventitial surface of a body passageway or cavity, which would allow drug concentrations to remain elevated for prolonged periods in regions where biological activity is most needed. For example paclitaxel may be delivered in a slow release form that contains from 0.001 mg/cm² to 5 mg/cm² (preferably 0.01 to 1.0 mg/cm²) over a selected period of time (*e.g.*, I to 120 days). For percutaneous administration, the agent may be administered at a dosage of 0.001 mg/ml to 30 mg/ml over a period of between 1 day and 90 days. In another embodiment of the disclosure, similar dose ranges may be used for sirolimus and analogues and derivatives, dactinomycin and analogues and derivatives, cervistatin and analogues and derivatives, 17-β-estradiol and analogues and derivatives, dexamethasone and analogues and derivatives, and doxorubicin and analogues and derivatives as examples of compound from the specific groupings described above. In yet another embodiment of the disclosure, the therapeutic agent, such as paclitaxel, may be placed in the appropriate location of a body cavity or a tumor resection site. For application at a tumor resection site, paclitaxel or other cell cycle inhibitor or microtubule stabilizing agent may be delivered in a slow release form that delivers from 0.01 mg/cm² to 20 mg/cm² mg/m² (preferably 0.01 to 10.0 mg/cm²) over a selected period of time (e.g., I to 150 days).

In another example, a patient undergoing balloon angioplasty has a sheath inserted into an artery that is to be catheterized (*e.g.*, femoral) and through which the guidewire and balloon angioplasty catheter will be introduced. The sheath remains in place throughout the procedure, oftentimes causing injury to the site of puncture. After the removal of the balloon angioplasty hardware, a needle would be inserted through the skin to the catheterization site and a therapeutic agent (*e.g.*, paclitaxel impregnated into a slow release polymer) or a polymer alone could be infiltrated through the needle or catheter in a circumferential manner directly around the catheterization site. This could be performed around any artery, vein, or graft, but ideal candidates for this intervention include procedures that require arterial and venous catheterization.

The following examples are offered by way of illustration, and not by way of limitation.

### EXAMPLES

### EXAMPLE 1

### SYNTHESIS OF POLYMER MEPEG750-PDLLA-2080 POLYMER

To synthesize the MePEG750-PDLLA-2080 polymer, 40 g of MePEG (molecular weight = 750; Sigma-Aldrich, St. Louis, MO) was weighed in a 500 RB flask and 160 g of D,L-lactide (PURASORB^{®}, PURAC, Lincolnshire, IL) was weighed in a weigh boat. Both reagents were dried under a vacuum overnight at room temperature. Then 600 mg stannous 2-ethyl-hexanoate catalyst (Sigma) was added into the RB flask containing the MePEG and a magnetic stir bar. The flask was purged with N₂ (oxygen free) for 5 minutes, capped with a glass stopper, placed into an oil-bath (maintained at 135°C), and a magnetic stirrer was gradually turned onto setting 6 (Coming). After 30 minutes, the flask was removed from the oil-bath and was cooled to room temperature in a water bath. The D,L-lactide was added into the flask, which was then purged with oxygen free N₂ for 15 minutes, the flask was capped and again placed in the oil-bath (135°C). The magnetic stirrer was turned on to a setting of 3 and the polymerization reaction was allowed to continue for at least five (5) hours. The flask was removed from the oil bath and the molten polymer poured into a glass container and allowed to cool to room temperature.

### EXAMPLE 2

### PURIFICATION OF MEPEG750-PDLLA-2080

The MePEG750-PDLLA-2080 was prepared as outlined in Example 1, then 75 g MePEG750-PDLLA-2080 was dissolved in 100 ml of ethyl acetate (Fisher, HPLC grade) in a 250 ml conical flask. The polymer was precipitated by slowly adding the solution into 900 ml isopropanol (Caledon, HPLC grade) in a 2 L conical flask while stirring. The solution was stirred for 30 minutes and the suspension cooled to 5°C using a cooling system. The supernatant was separated and the precipitant transferred to a 400 ml beaker. The polymer was first pre-dried in a forced-air oven at 50°C for 24 hours to remove the bulk of the solvent. The pre-dried polymer was then transferred to a vacuum oven (50°C) and further dried for 24 hours until the residual solvent was below an acceptable level. The purified polymer was stored at 2-8°C.

### EXAMPLE 3

### COATING OF MEPEG750-PDLLA-2080 ON A PLGA (10:90) MESH

A PLGA (10/90) mesh of dimension 3 x 6 cm² was washed with isopropanol (Caledon, HPLC) and dried in, a forced-air oven at 50°C. Then 3 g MePEG750-PDLLA-2080 was dissolved in 15 ml ethyl acetate (20% solution; Fisher HPLC grade) in a 20 mL glass scintillation vial. Paclitaxel (10.13 mg) was added to the polymer solution and the paclitaxel was completely dissolved by using a vortex mixer. A mesh was coated with the polymer/paclitaxel solution by dipping into such a solution. The excess solution was then removed and the coated mesh was dried using an electric fan for 2-3 minutes. The coated mesh was placed in a PTFE petri-dish and was further dried for 60 minutes using the electric fan in a fume-hood. The coated mesh was then transferred into a vacuum oven and dried under vacuum overnight at room temperature. The dried coated mesh was packed between two pieces of release-liners (Rexam A10) and sealed in a pouch bag.

### EXAMPLE 4

### IN VITRO RELEASE PROFILE OF PACLITAXEL FROM A MESH

Mesh samples were coated with PLGA (50:50, IV=0.15dL/g) in a similar manner to that described in this example.

### Release Studies

The release profile of paclitaxel was determined using an in-vitro release method. A portion of the mesh was sampled by cutting a sample piece, weighing the sample (approx. 5-7 mg), and placing in a screw top culture tube (16x125mm, Kimax). Then a phosphate/albumin buffer (15 mL) was added to the culture tube. The samples were placed on a rotating disk [30 rpm, 20° incline] (Fisher, Plate) in an incubator (VWR, Model 1380 Forced Air Oven) that was set at 37°C. After a specific incubation period, the culture tubes were removed from the incubation oven, the buffer was transferred to a second culture tube using a pipette, 15 mL of a phosphate/albumin buffer was added to the original mesh sample tube and the culture tubes were returned to the rotating disk in the incubation oven. The buffer was exchanged 3 times during the initial 24 hours, exchanged daily for the next 4 days and then exchanged on Mondays, Wednesdays and Fridays until the release study was completed.

### Extraction of Paclitaxel from the release buffer

Dichloromethane (1 mL) was added to 14 ml of paclitaxel-containing buffer. The tubes were vigorously shaken by hand for 10 seconds and then placed on a tube rotator (Thermolyne Labquake Shaker) for 15 minutes. The samples were centrifuged at 1500 rpm for 10 min. The supernatant buffer was withdrawn from the culture tube and the samples were then placed in a heating block (Pierce, ReactiTherm/ReactiVap) at 45°C. The samples were dried using a stream of nitrogen. The culture tubes that contained the dried samples were capped and placed in a -20°C freezer until HPLC analysis of the samples could be performed.

### Determination of Paclitaxel content by HPLC

An acetonitrile/water solution [50:50] was added (1 mL) to the culture tube containing the dried extract. The samples were vortexed for 60 sec on a vortexer (XXX). The samples were centrifuged for 15 min at 1500 rpm. Approx. 500 uL of the supernatant was transferred to an Agilent HPLC autosampler vial. The chromatographic conditions used for the determination of the paclitaxel content were : Solvent: water/ACN 47:53, Column: Hypersil ODS 125 x 4 mm, 5 um (Agilent), flow: 1mL/min, UV detection @ 232 nm, Gradient: isocratic, runtime: 5 min, injection volume: 10 uL. An external calibration curve using paclitaxel and 7-epipaclitaxel was used to quantify the paclitaxel in the extracts. The release profile of paclitaxel from the samples was plotted as percent pacltiaxel release against time.

### EXAMPLE 5

### EVALUATION OF PACLITAXEL CONTAINING MESH ON INTIMAL HYPERPLASIA DEVELOPMENT IN A RAT BALLOON INJURY CAROTID ARTERY MODEL

Rat balloon injury carotid artery model was used to demonstrate the efficacy of a paclitaxel containing mesh system on the development of intimal hyperplasia fourteen days following placement.

### Control Group

Wistar rats weighing 400 - 500 g were anesthetized with 1.5% halothane in oxygen and the left external carotid artery was exposed. An A 2 French Fogarty balloon embolectomy catheter (Baxter, Irvine, CA) was advanced through an arteriotomy in the external carotid artery down the left common carotid artery to the aorta. The balloon was inflated with enough saline to generate slight resistance (approximately 0.02 ml) and it was withdrawn with a twisting motion to the carotid bifurcation. The balloon was then deflated and the procedure repeated twice more. This technique produced distension of the arterial wall and denudation of the endothelium. The external carotid artery was ligated after removal of the catheter. The right common carotid artery was not injured and was used as a control.

### Local Perivascular Paclitaxel Treatment

Immediately after injury of the left common carotid artery, a 1 cm long distal segment of the artery was exposed and treated with a 1x1 cm paclitaxel-containing mesh. The wound was then closed the animals were kept for 14 days.

### Histology and immunohistochemistry

At the time of sacrifice, the animals were euthanized with carbon dioxide and pressure perfused at 100 mmHg with 10% phosphate buffered formaldehyde for 15 minutes. Both carotid arteries were harvested and left overnight in fixative. The fixed arteries were processed and embedded in paraffin wax. Serial cross-sections were cut at 3 µm thickness every 2 mm within and outside the implant region of the injured left carotid artery and at corresponding levels in the control right carotid artery. Cross-sections were stained with Mayer's hematoxylin-and-eosin for cell count and with Movat's pentachrome stains for morphometry analysis and for extracellular matrix composition assessment.

### Results

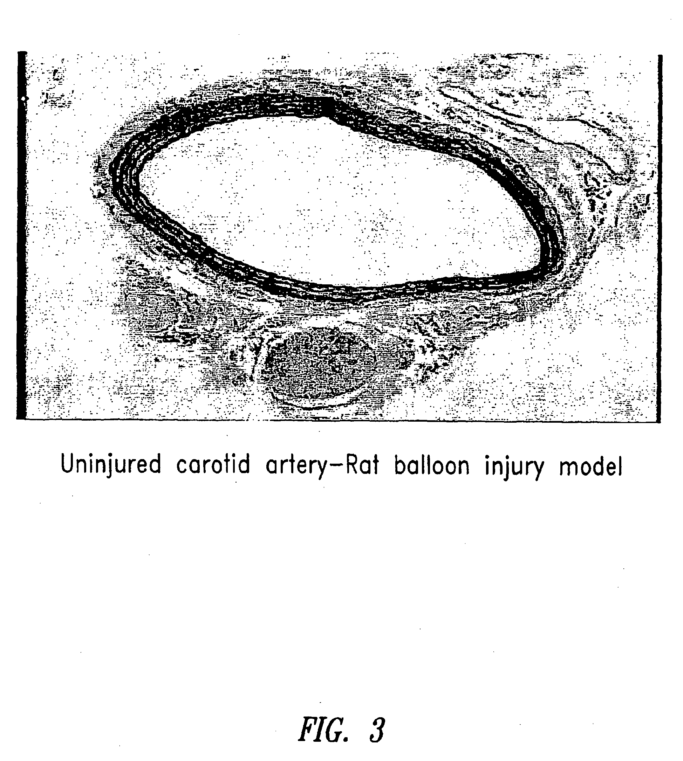

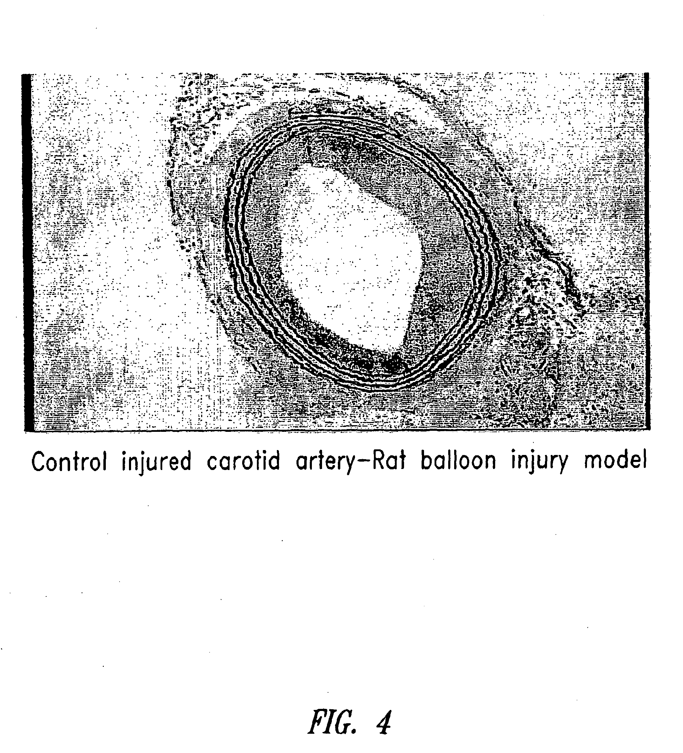

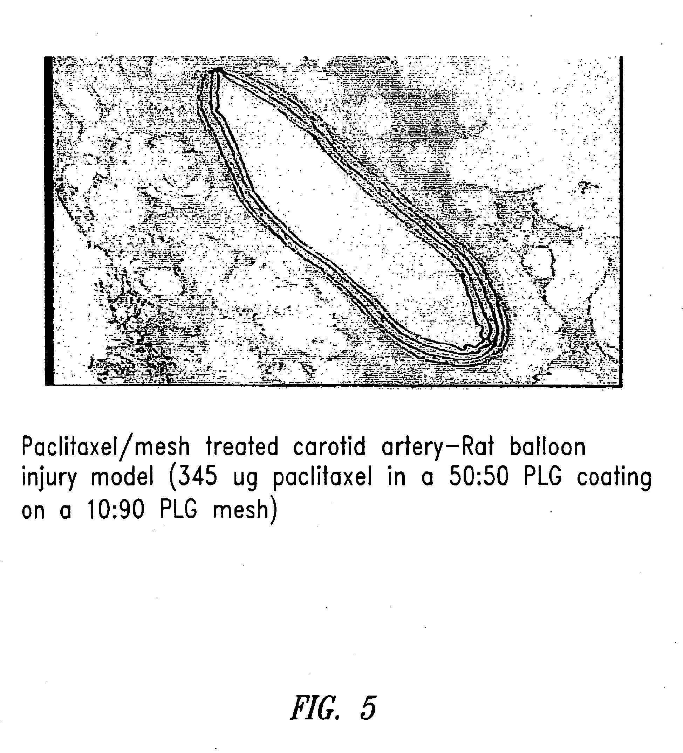

From Figures 3-5, it is evident that the perivascular delivery of paclitaxel using the paclitaxel.mesh formulation resulted is a dramatic reduction in intimal hyperplasia.

### EXAMPLE 6

### EVALUATION OF PACLITAXEL CONTAINING MESH ON INTIMAL HYPERPLASIA DEVELOPMENT IN A SHEEP CAROTID ARTERY BYPASS GRAFT MODEL

Expanded polytetrafluoroethylene (ePTFE) is the most common material used for prosthetic vascular grafts, but the majority of these grafts fail over time, usually because of stenosis at the distal anastomosis site due to development of intimal hyperplasia.

The objective of this study was evaluation of the extent of intimal hyperplasia formation following use of a biodegradable, bioresorbable mesh containing paclitaxel and placed at the ePTFE distal anastomosis site. Paclitaxel is a drug that inhibits processes important in intimal hyperplasia development, including without limitation, inhibition of smooth muscle cell proliferation, cell migration, and matrix deposition.

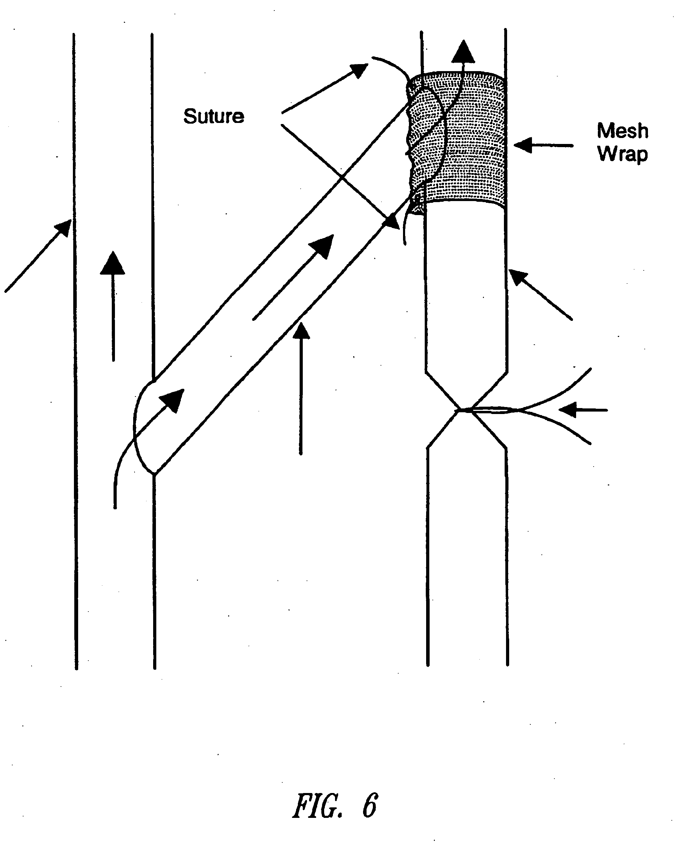

The left and right carotid arteries of anesthetized sheep were exposed by sharp dissection. A tunnel was formed from one carotid artery to the other to accommodate the ePTFE graft. The ePTFE graft was tunneled and trimmed for appropriate length and configuration. Using standard vascular technique, the ePTFE graft was anastomosed end-to-side with running 6-0 polypropylene suture. The angle of the junction between graft and native vessel was approximately 45°. The length of the implanted graft ranged from 9.5 - 15 cm (average 11 cm). The graft implant configuration is illustrated in Figure 6.

Paclitaxel was incorporated into the 2 cm x 5 cm PLG mesh in the following doses and animal test groups: Group 1, 0 mg; Group 2, 0.6 mg; Group 3, 1.8 mg; and Group 4, 3.0 mg. The mesh was placed at the distal end of the graft at the anastomosis site. To place the mesh, the long side was pulled under the artery and up around either side of the distal end of the graft. One edge was positioned as close to the heel of the anastomosis as possible. The top edges of the mesh were sewn together with one suture on either side of the graft so that no gaps were left in the circumferential direction. One suture was placed at the proximal end and the other at the distal end of the mesh, and sewn to nearby connective tissue to prevent slippage of the mesh away from the anastomosis (*see* Figure 6). The surgical sites were closed in layers with running absorbable sutures. Standard antibiotics and analgesics were administered after surgery for several days as required.

At approximately 56 days post-graft implant, animals were anesthetized. Contrast media was injected and angiograms performed of the distal graft and artery at the distal anastomosis. Immediately prior to euthanasia, the animals received heparin (150 U/kg, IV) and immediately after euthanasia, the ePTFE graft was rinsed *in situ* with lactated Ringers solution and perfusion-fixed *in situ* with 10% neutral buffered formalin (NBF). The specimens were excised *en bloc* and allowed to immersion fix in 10% NBF at least 24 hours prior to histological processing.

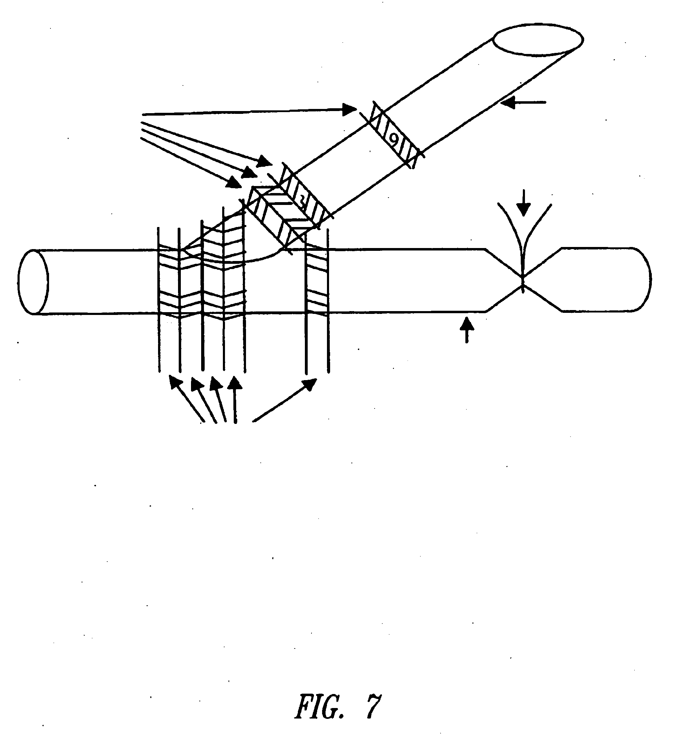

The fixed specimens were trimmed and mapped accurately for corresponding cross sectional location in reference to the ePTFE graft configuration. The scheme for sectioning is illustrated in Figure 7. A total of nine sections were cut at the distal end of the graft: two cut perpendicular to the artery on either side of the anastomosis (A 1 and A5), one perpendicular to the artery through the "toe" of the anastomosis (A2), one or two cut through the floor of the anastomosis adjacent to the "toe" (A3 and A4), three cut perpendicular to the graft at its distal end, and one through the center of the graft. Adjacent sections were cut at approximately 3 mm intervals. The specimens were paraffin-embedded, cross-sectioned, and four sets of slides made, two stained with hematoxylin and eosin (H&E), and one each stained with Masson's trichrome and Verhoeff Van Gieson (VVG). These stains were selected for their ability to show tissue cellularity (H&E), collagen, smooth muscle and fibrin (Masson's Trichrome), and elastin (VVG).

### Morphometric Analysis:

The morphometric analysis system consists of an Olympus BX40 microscope, Optronics Image Sensor DEI-750, Sony HR Trinitron monitor, and PC computer equipped with Media Cybernetics Image-Pro Plus software v. 3.0 for Windows. Digital images are created, labeled, and stored according to applicable BioDevelopment Associates SOPs. With regard to the results, the following definitions apply: Proximal- toward the heart; Distal- away from the heart; Anastomosis- surgical connection of graft to native vessel; "Toe" of Anastomosis- where graft and vessel meet at an obtuse angle; "Heel" of Anastomosis- where graft and vessel meet at an acute angle; "Floor" of Anastomosis- region between toe and heal; Stenosis- narrowing of graft or vessel lumen; Neointima- hyperplastic lesion on luminal surface characterized by proliferating smooth muscle cells (SMC); Pseudointima- lesion on luminal surface composed of aged thrombus, which is not undergoing typical reorganization by SMC proliferation.

Morphometric measurements of histological cross sections included neointimal area (IA), maximal neointimal thickness (MIT), luminal area (LA), and area inside the graft (GA). GA = IA + LA. Area inside the graft was the reference measurement from which stenosis was determined (percent stenosis 100*IA/GA). In asymmetrical sections through the floor of the anastomosis, where graft sections were not complete, only MIT was measured.

Morphometric analysis was performed on sections A2 ("toe" section cut perpendicular to the native vessel), and on sections A6, A7 and A8 (the first three complete graft sections cut perpendicular to the graft at it's distal end) (*see* Figure 7). Group results were compared using a one-tailed t-test. Each of the paclitaxel mesh groups was compared to the zero-dose mesh group. A summary presentation of group morphometric data is shown in Tables 1-3. Group averages for all parameters in all sections in all paclitaxel groups were less than corresponding data from the zero-dose controls.

Intraluminal lesions that represented permanent or semi-permanent luminal obstructions and thus contributed functionally to reduction in blood flow were included in the morphometric analysis. Both neointima (hyperplastic lesion characterized by proliferating SMC) and pseudointima (aged adherent thrombus not undergoing typical reorganization by SMC migration and proliferation) were included in the analysis, whereas fresh thrombus was not. In reporting the morphometric data, no distinction was made between neointima and pseudointima since both represented stenotic lesions.

The MIT in Section 2 ("toe" section) for Group 1 (controls) was 0.82 ± 0.29 mm (group average ± SD). The low, mid, and high dose paclitaxel groups had values of 0.78 ± 0.30 mm, 0.59 ± 0.14, mm and 0.54 ± 0.23 mm, respectively (5%, 28%, and 34% less than controls), but these differences were not statistically significant at a 95% confidence interval (p>0.05). MIT in section 6 (first full cross section of graft adjacent to the distal anastomosis) in the controls was 1.31 ± 0.82 mm. The low, medium, and high dose paclitaxel groups had MIT in section 6 of 0.38 ± 0.12 mm, 0.31 ± 0.29 mm, and 0.34 ± 0.20 mm, respectively. The reductions in MIT in Groups 1, 2 and 3 were statistically significant (p ≤ 0.05). In sections 7 and 8 (approximately 3 mm and 6 mm past section 6), MIT in the controls was 0.95 ± 0.67 mm and 0.89 ± 0.64 mm, respectively. Although MIT in sections 7 and 8 in all the paclitaxel groups was approximately 70% less than controls, only two values, section 7 Group 3 and section 8 Group 4, were statistically significant (p ≤ 0.05).

The IA of the control group was 7.41 ± 5.12 mm, 6.28 ± 4.31 mm, and 5.57 ± 4.62 mm in sections 6, 7, and 8, respectively. In the paclitaxel groups, IA was reduced approximately 70-80%. Reductions in IA for section 6 in Groups 3 and 4 and for section 7 in Group 2, 3 and 4 were statistically significant (p ≤ 0.05).

The percent stenosis due to neointima in the control group in section 6 was 28.4 ± 19.5 mm². As was the case for the other parameters, stenosis did not decrease markedly at sites 3 and 6 mm into the graft from the anastomosis. Likewise, the effect of paclitaxel on reducing stenosis was similar to the effect on IA, with approximately 70-80% reduction in stenosis, and 7 of 9 values were significantly lower than controls (p ≤ 0.05).

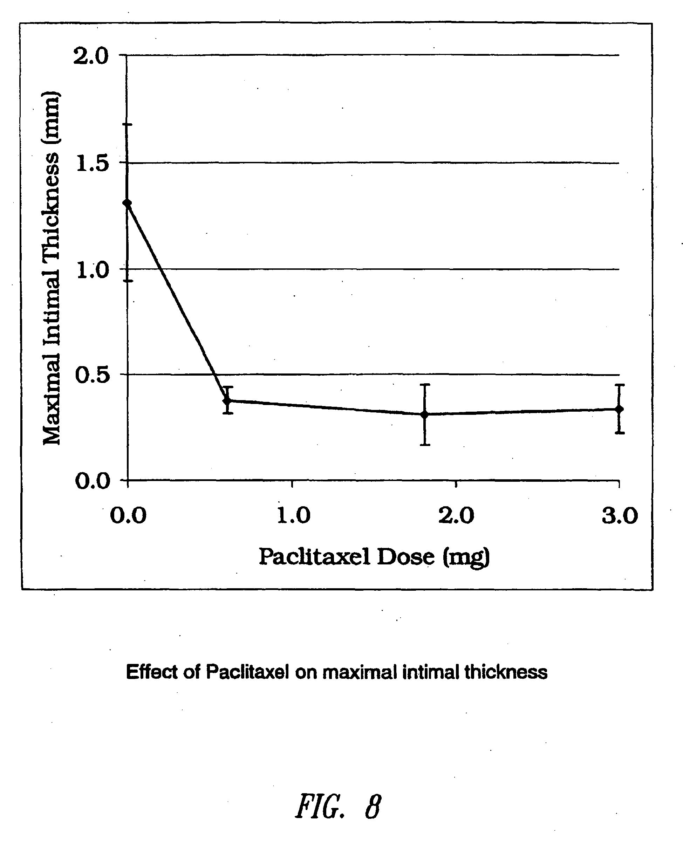

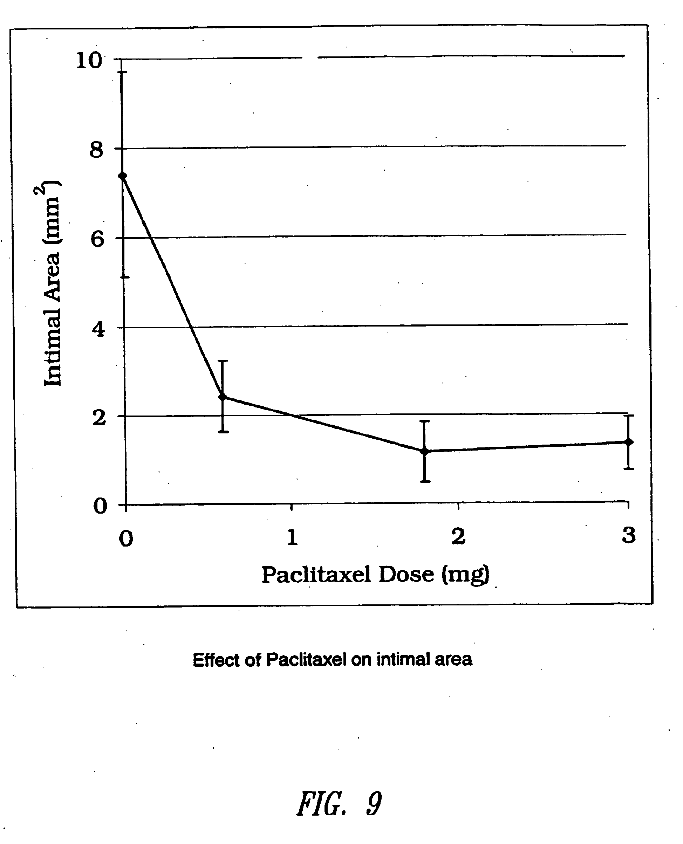

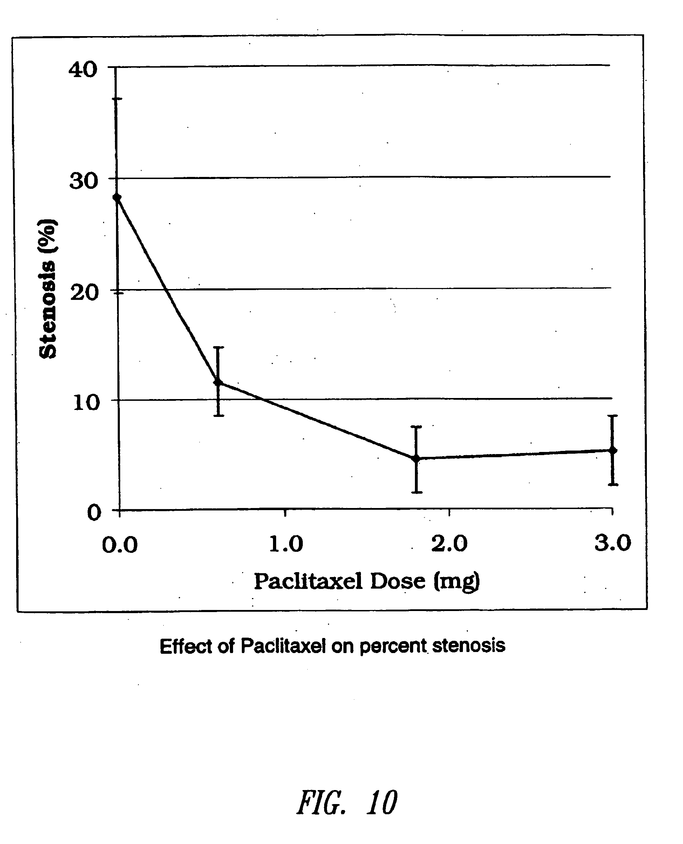

There did not appear to be a marked dose effect of paclitaxel on luminal lesions (neointima and/or pseudointima) that contributed to stenosis. Figures 8-10 clearly illustrate this point. There is an indication that stenosis was reduced slightly more at the mid paclitaxel dose than the low dose, but clearly there is no further gain in efficacy at the high dose.

The attrition rate in this study due to early graft occlusion was larger than expected at the outset. The attrition rate appeared to have a dose dependence, which is supported by the histopathology analysis. At the lowest paclitaxel dose, 0.6 mg, there was a marked and significant reduction in lesions causing luminal narrowing at the distal end of the graft. This effect did not increase markedly with increased dose, suggesting that the low dose achieved near maximal response in terms of efficacy to inhibit stenosis. The inhibitory effect of paclitaxel does not affect the mechanical integrity of the anastomosis (no evidence of leakage) in the dose range tested. Intraluminal endothelialization is not affected by paclitaxel. Finally, paclitaxel in the doses tested is not toxic to the native artery wall. Thus, the results of this study suggest that low and mid doses represent a useful clinical range of efficacy and safety.

### EFFECT OF PACLITAXEL ON INTIMAL HYPERPLASIA, SUMMARY OF RESULTS

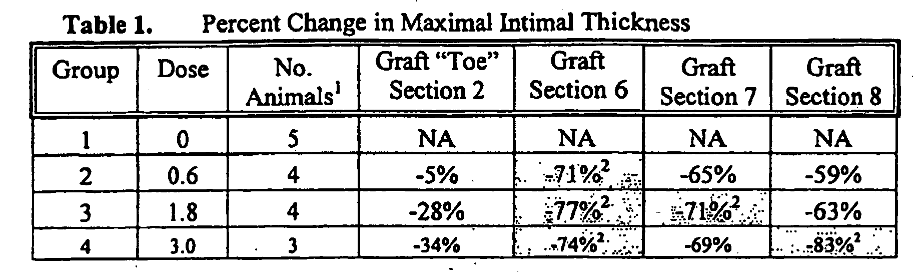

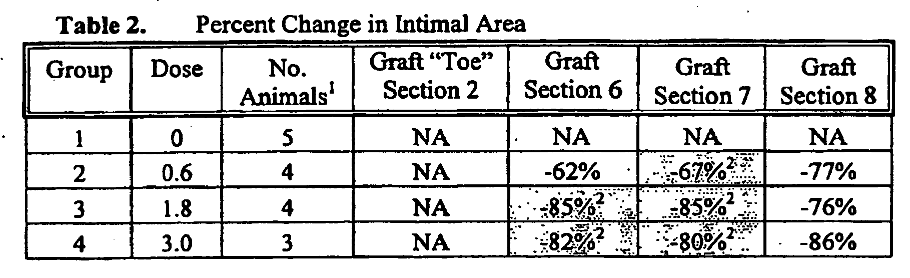

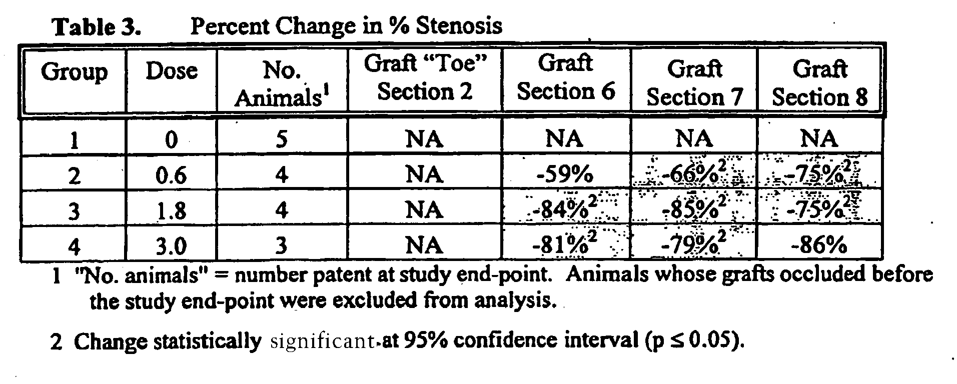

From the foregoing it will be appreciated that, although specific embodiments of the invention have been described herein for purposes of illustration, various modifications may be made without deviating from the invention. Accordingly, the invention is not limited except as by the appended claims.

## Claims

1. A perivascular wrap comprising: (1) a biodegradable polymer, (2) a therapeutic agent, and (3) a polymeric carrier of the therapeutic agent, wherein the biodegradable polymer is in the form of a mesh, and the polymeric carrier of the therapeutic agent comprises a diblock or triblock copolymer.

2. The perivascular wrap of claim 1 wherein the biodegradable polymer is formed from one or more monomers selected from the group consisting of lactide, glycolide, ε-caprolactone, trimethylene carbonate, 1,4-dioxan-2-one, 1,5-dioxepan-2-one, 1,4-dioxepan-2-one, hydroxyvalerate, and hydroxybutyrate.

3. The perivascular wrap of claim 1 wherein the biodegradable polymer is a poly(caprolactone), a poly(lactic acid), or a copolymer of lactide and ε-caprolactone.

4. The perivascular wrap of claim 1 wherein the biodegradable polymer comprises a polyester.

5. The perivascular wrap of claim 1 wherein the biodegradable polymer comprises a poly(lactide-co-glycolide).

6. The perivascular wrap of claim 5 wherein the poly(lactide-co-glycolide) is a poly(L-lactide-co-glycolide).

7. The perivascular wrap of claim 5 or claim 6 wherein the poly(lactide-co-glycolide) has a lactide:glycolide molar ratio range from 3:97 to 15:85.

8. The perivascular wrap of any one of the preceding claims wherein the therapeutic agent is an anti-angiogenic agent.

9. The perivascular wrap of claim 8 wherein the anti-angiogenic agent is paclitaxel, fucoidon, doxorubicin, or an analogue or derivative thereof.

10. The perivascular wrap of claim 8 wherein the anti-angiogenic agent is paclitaxel.

11. The perivascular wrap of any one of claims 1 to 7 wherein the therapeutic agent is capable of inhibiting smooth muscle cell migration, proliferation, matrix production, inflammation, or a combination thereof.

12. The perivascular wrap of any one of claims 1 to 7 wherein the therapeutic agent is an anti-inflammatory agent.

13. The perivascular wrap of any one of claims 1 to 7 wherein the therapeutic agent is an antibiotic neoplastic agent.

14. The perivascular wrap of any one of claims 1 to 7 wherein the therapeutic agent is an estrogen.

15. The perivascular wrap of any one of claims 1 to 7 wherein the therapeutic agent is an antibacterial agent, an antifungal agent, or an antiviral agent.

16. The perivascular wrap of any one of claims 1 to 7 wherein the therapeutic agent is an immunosuppressive antibiotic.

17. The perivascular wrap of claim 10 comprising 0.001 mg/cm² to 5 mg/cm² of paclitaxel.

18. The perivascular wrap of claim 10 comprising 0.03 mg/cm² to 0.3 mg/cm² of paclitaxel.

19. The perivascular wrap of any one of claims 1-18 wherein the polymeric carrier is biodegradable.

20. The perivascular wrap of claim 19 wherein the biodegradable polymeric carrier comprises a diblock copolymer having a first block and a second block, wherein the first block comprises methoxypoly(ethylene glycol) and the second block comprises a polyester.

21. The perivascular wrap of claim 20 wherein the polyester is a poly(lactide), poly(glycolide), poly(caprolactone), trimethylene carbonate polymer, poly(hydroxyl acid), poly(D,L-lactide-co-glycolide), poly(L-lactide-co-glycolide), copolymer of lactic acid and glycolic acid, copolymer of ε-caprolactone and lactide, copolymer of glycolide and ε-caprolactone, or a copolymer of lactide and 1,4-dioxane-2-one.

22. The perivascular wrap of claim 20 wherein the polyester is a polymer or copolymer comprising one or more of the residue units of the monomers selected from D-lactide, L-lactide, glycolide, ε-caprolactone, trimethylene carbonate, 1,4-dioxane-2-one, 1,5-dioxepan-2-one, hydroxyvalerate, and hydroxybutyrate.

23. The perivascular wrap of claim 20 wherein the polyester is formed from one or more monomers selected from the group consisting of lactide, glycolide, ε-caprolactone, trimethylene carbonate, 1,4-dioxan-2-one, 1,5-dioxepan-2-one, hydroxyvalerate, and hydroxybutyrate.

24. The perivascular wrap of claim 21 wherein the poly(lactide) is poly(D,L-lactide).

25. The perivascular wrap of claim 20 wherein the diblock copolymer has a methoxypoly(ethylene glycol) : polyester ratio in the range of 10:90 (± 15%) to 30:70 (± 15%) by weight.

26. The perivascular wrap of claim 20 wherein the diblock copolymer has a methoxypoly(ethylene glycol):polyester ratio of 20:80 (± 15%) by weight.

27. The perivascular wrap of claim 24 wherein the methoxypoly(ethylene glycol):poly (D,L-lactide) ratio is 20:80 (± 15%) by weight.

28. The perivascular wrap of any one of claims 20-27 wherein the methoxypoly(ethylene glycol) has a molecular weight range of 200 g/mol (± 15%) to 5000 g/mol (±15%).

29. The perivascular wrap of claim 28 wherein the methoxypoly(ethylene glycol) has a molecular weight of 750 g/mol (± 15%).

30. The perivascular wrap of claim 19 wherein the biodegradable polymeric carrier comprises a triblock copolymer comprising a structure of A-B-A or B-A-B, wherein the A block comprises polyoxyalkane, and the B block comprises a polyester.

31. The perivascular wrap of claim 30 wherein the polyoxyalkane is a poly(ethylene glycol), a poly(ethylene oxide-co-propylene oxide), or a poly(ethylene oxide-co-propylene oxide-co-ethylene oxide).

32. The perivascular wrap of claim 30 wherein the polyester is a poly(lactide), poly(glycolide), poly(caprolactone), trimethylene carbonate polymer, poly(hydroxyl acids), poly(D,L-lactide-co-glycolide), poly(L-lactide-co-glycolide), copolymer of lactic acid and glycolic acid, copolymer of ε-caprolactone and lactide, copolymer of glycolide and ε-caprolactone, or a copolymer of lactide and 1,4-dioxane-2-one.

33. The perivascular wrap of claim 30 wherein the polyester is a polymer or copolymer comprising one or more of the residue units of monomers selected from D-lactide, L-lactide, D,L-lactide, glycolide, ε-caprolactone, trimethylene carbonate, 1,4-dioxane-2-one, and 1,5-dioxepan-2-one.

34. The perivascular wrap of claim 30 wherein the polyester is formed from one or more monomers selected from the group consisting of lactide, glycolide, ε-caprolactone, trimethylene carbonate, 1,4-dioxan-2-one, 1,5-dioxepan-2-one, 1,4-dioxepan-2-one, hydroxyvalerate, and hydroxybutyrate.

35. The perivascular wrap of claim 1 wherein the biodegradable polymer is poly(lactide-co-glycolide), the therapeutic agent is paclitaxel, the polymeric carrier of the therapeutic agent is a diblock polymer having a first block that comprises methoxypoly(ethylene glycol) and a second block that comprises poly(D,L-lactide), and the perivascular wrap comprises 0.03 mg/cm² to 0.3 mg/cm² of paclitaxel.

36. The perivascular wrap of any one of claims 1 to 35 wherein the therapeutic agent and the polymeric carrier of the therapeutic agent form a coating on the biodegradable polymer.

37. A method for producing a perivascular wrap of claim 36 comprising coating or attaching directly a biodegradable polymer in the form of a mesh with a composition comprising a therapeutic agent and a diblock or triblock carrier of the therapeutic agent.

38. The method of claim 37 comprising coating the biodegradable polymer with the composition comprising the therapeutic agent and the diblock or triblock carrier of the therapeutic agent.

39. The method of claim 38 wherein the biodegradable polymer is coated by painting with the composition comprising the therapeutic agent and the diblock or triblock carrier of the therapeutic agent.

40. The method of claim 38 wherein the biodegradable polymer is coated by dipping into the composition comprising the therapeutic agent and the diblock or triblock carrier of the therapeutic agent.

41. The method of claim 38 wherein the biodegradable polymer is coated by spraying with the composition comprising the therapeutic agent and the diblock or triblock carrier of the therapeutic agent.

42. The method of claim 37 comprising attaching directly the biodegradable polymer with the composition comprising the therapeutic agent and the diblock or triblock carrier of the therapeutic agent.

## Patentansprüche

1. Eine perivaskuläre Hülle, die umfasst: (1) ein biologisch abbaubares Polymer, (2) ein Therapeutikum und (3) einen Polymerträger des Therapeutikums, wobei das biologisch abbaubare Polymer in der Form eines Netzes ist und der Polymerträger des Therapeutikums ein Diblock- oder Triblock-Copolymer umfasst.

2. Die perivaskuläre Hülle nach Anspruch 1, wobei das biologisch abbaubare Polymer aus einem oder mehreren Monomeren gebildet wird, die aus der Gruppe, die aus Laktid, Glycolid, ε-Caprolacton, Trimethylencarbonat, 1,4-Dioxan-2-on, 1,5-Dioxepan-2-on, 1,4-Dioxepan-2-on, Hydroxyvalerat und Hydroxybutyrat besteht, ausgewählt werden.

3. Die perivaskuläre Hülle nach Anspruch 1, wobei das biologisch abbaubare Polymer ein Poly(caprolacton), eine Poly(milchsäure) oder ein Copolymer aus Lactid und ε-Caprolacton ist.

4. Die perivaskuläre Hülle nach Anspruch 1, wobei das biologisch abbaubare Polymer ein Polyester umfasst.

5. Die perivaskuläre Hülle nach Anspruch 1, wobei das biologisch abbaubare Polymer ein Poly(lactid-co-glycolid) umfasst.

6. Die perivaskuläre Hülle nach Anspruch 5, wobei das Poly(lactid-co-glycolid) ein Poly(L-lactid-co-glycolid) ist.

7. Die perivaskuläre Hülle nach Anspruch 5 oder Anspruch 6, wobei das Poly(lactid-co-glycolid) ein Lactid : Glycolid Molverhältnis im Bereich von 3:97 bis 15:85 hat.

8. Die perivaskuläre Hülle nach einem der vorangegangenen Ansprüche, wobei das Therapeutikum ein anti-angiogenes Mittel ist.

9. Die perivaskuläre Hülle nach Anspruch 8, wobei das anti-angiogenes Mittel Paclitaxel, Fucoidon, Doxorubicin oder ein Analogon oder ein Derivat davon ist.

10. Die perivaskuläre Hülle nach Anspruch 8, wobei das antiangiogene Mittel Paclitaxel ist.

11. Die perivaskuläre Hülle nach einem der Ansprüche 1 bis 7, wobei das Therapeutikum die Migration von glatten MuskelZellen, Proliferation, Matrixherstellung, Entzündung oder eine Kombination davon inhibieren kann.

12. Die perivaskuläre Hülle nach einem der Ansprüche 1 bis 7, wobei das Therapeutikum ein entzündungshemmendes Mittel ist.

13. Die perivaskuläre Hülle nach einem der Ansprüche 1 bis 7, wobei das Therapeutikum ein antibiotisches, neoplastisches Mittel ist.

14. Die perivaskuläre Hülle nach einem der Ansprüche 1 bis 7, wobei das Therapeutikum ein Östrogen ist.

15. Die perivaskuläre Hülle nach einem der Ansprüche 1 bis 7, wobei das Therapeutikum ein antibakterielles Mittel, ein Antipilzmittel oder ein antivirales Mittel.

16. Die perivaskuläre Hülle nach einem der Ansprüche 1 bis 7, wobei das Therapeutikum ein immunosuppresives Antibiotikum ist.

17. Die perivaskuläre Hülle nach Anspruch 10, die 0.001 mg/cm² bis 5 mg/cm² Paclitaxel umfasst.

18. Die perivaskuläre Hülle nach Anspruch 10, die 0.03 mg/cm² bis 0.3 mg/cm² Paclitaxel umfasst.

19. Die perivaskuläre Hülle nach einem der Ansprüche 1-18, wobei der Polymerträger biologisch abbaubar ist.

20. Die perivaskuläre Hülle nach Anspruch 19, wobei der biologisch abbaubare Polymerträger ein Diblock-Copolymer mit einem ersten Block und einem zweiten Block umfasst, wobei der erste Block Methoxypoly(ethylenglycol) umfasst und der zweite Block ein Polyester umfasst.

21. Die perivaskuläre Hülle nach Anspruch 20, wobei das Polyester ein Poly(lactid), ein Poly(glycolid), ein Poly(caprolacton), ein Trimethylencarbonat-Polymer, eine Poly(hydroxylsäure), ein Poly(D,L-lactid-co-glycolid), ein Poly(L-lactid-co-glycolid), ein Copolymer aus Milchsäure und Glycolsäure, ein Copolymer aus ε-Caprolacton und Lactid, ein Copolymer aus Glycolid und ε-Caprolacton oder ein Copolymer aus Milchsäure und 1,4-Dioxan-2-on ist.

22. Die perivaskuläre Hülle nach Anspruch 20, wobei das Polyester ein Polymer oder Copolymer ist, das eine oder mehrere der Resteinheiten der Monomere umfasst, die aus D-Lactid, L-Lactid Glycolid, ε-Caprolacton, Trimethylencarbonat, 1,4-Dioxan-2-on, 1,5-Dioxepan-2-on, Hydroxyvalerat und Hydroxybutyrat ausgewählt werden.

23. Die perivaskuläre Hülle nach Anspruch 20, wobei das Polyester aus einem oder mehreren Monomeren gebildet wird, die aus der Gruppe, die aus Lactid, Glycolid, ε-Caprolacton, Trimethylencarbonat, 1,4-Dioxan-2-on, 1,5-Dioxepan-2-on, Hydroxyvalerat und Hydroxybutyrat besteht, ausgewählt werden.

24. Die perivaskuläre Hülle nach Anspruch 21, wobei das Poly(lactid) Poly(D,L-lactid) ist.

25. Die perivaskuläre Hülle nach Anspruch 20, wobei das Diblock-Copolymer ein Methoxypoly(ethylenglycol) Polyester Gewichtsverhältnis im Bereich von 10:90 (± 15%) bis 30:70 (± 15%) hat.

26. Die perivaskuläre Hülle nach Anspruch 20, wobei das Diblock-Copolymer ein Methoxypoly(ethylenglycol) : Polyester Gewichtsverhältnis im Bereich von 20:80 (± 15%) hat.

27. Die perivaskuläre Hülle nach Anspruch 24, wobei das Methoxypoly(ethylenglycol) : Poly(D,L-lactid) Gewichtsverhältnis 20:80 (± 15%) ist.

28. Die perivaskuläre Hülle nach einem der Ansprüche 20-27, wobei das Methoxypoly(ethylenglycol) einen Molekulargewichtsbereich von 200 g/ml 20:80 (± 15%) bis 5000 g/mol (± 15%) hat.

29. Die perivaskuläre Hülle nach Anspruch 28, wobei das Methoxypoly(ethylenglycol) ein Molekulargewicht von 750 g/mol (± 15%) hat.

30. Die perivaskuläre Hülle nach Anspruch 19, wobei der biologisch abbaubare Polymerträger ein Triblock-Copolymer umfasst, das eine Struktur A-B-A oder B-A-B umfasst, wobei der A-Block Polyoxyalkan umfasst und der B-Block ein Polyester umfasst.

31. Die perivaskuläre Hülle nach Anspruch 30, wobei das Polyoxyalkan ein Poly(ethylenglycol), ein Poly(ethylenoxid-co-propylenoxid) oder ein Poly(ethylenoxid-co-propylenoxid-co-ethylenoxid) ist.

32. Die perivaskuläre Hülle nach Anspruch 30, wobei das Polyester ein Poly(lactid), ein Poly(glycolid), ein Poly(caprolacton), ein Trimethylencarbonat-Polymer, eine Poly(hydroxylsäure), ein Poly(D,L-lactid-co-glycolid), ein Poly(L-lactid-co-glycolid), ein Copolymer aus Milchsäure und Glycolsäure, ein Copolymer aus ε-Caprolacton und Lactid, ein Copolymer aus Glycolid und ε-Caprolacton oder ein Copolymer aus Milchsäure und 1,4-Dioxan-2-on ist.

33. Die perivaskuläre Hülle nach Anspruch 30, wobei das Polyester ein Polymer oder Copolymer ist, das einen oder mehrere Resteinheiten von Monomeren umfasst, die aus D-Lactid, L-Lactid, D,L-Lactid, Glycolid, ε-Caprolacton, Trimethylencarbonat, 1,4-Dioxan-2-on und 1,5-Dioxepan-2-on ausgewählt werden.

34. Die perivaskuläre Hülle nach Anspruch 30, wobei das Polyester aus einem oder mehreren Monomeren gebildet wird, die aus der Gruppe, die aus Lactid, Glycolid, ε-Caprolacton, Trimethylencarbonat, 1,4-Dioxan-2-on, 1,5-Dioxepan-2-on, 1,4-Dioxepan-2-on, Hydroxyvalerat und Hydroxybutyrat besteht, ausgewählt werden.

35. Die perivaskuläre Hülle nach Anspruch 1, wobei das biologisch abbaubare Polymer Poly(lactid-co-glycolid) ist, das Therapeutikum Paclitaxel ist, der Polymerträger des Therapeutikums ein Diblock-Polymer mit einem ersten Block, der Methoxypoly(ethylenglycol) umfasst und einem zweiten Block, der Poly(D,L-lactid) umfasst, ist und die perivaskuläre Hülle 0.03 mg/cm² bis 0.3 mg/cm² Paclitaxel umfasst.

36. Die perivaskuläre Hülle nach einem der Ansprüche 1 bis 35, wobei das Therapeutikum und der Polymerträger des Therapeutikums eine Beschichtung auf dem biologisch abbaubaren Polymer bilden.

37. Ein Verfahren zur Herstellung einer perivaskulären Hülle nach Anspruch 36, das das Beschichten oder direkte Anbringen eines biologisch abbaubaren Polymers in Form eines Netzes mit einer Zusammensetzung, die ein Therapeutikum und einen Diblock- oder Triblockträger des Therapeutikums umfasst, umfasst.

38. Das Verfahren nach Anspruch 37, dass das Beschichten des biologisch abbaubaren Polymers mit der Zusammensetzung umfasst, die das Therapeutikum und den Diblock- oder Triblockträger des Therapeutikums umfasst.

39. Das Verfahren nach Anspruch 38, wobei das biologisch abbaubare Polymer durch Bestreichen mit der Zusammensetzung, die das Therapeutikum und den Diblock-oder Triblockträger des Therapeutikums umfasst, beschichtet wird.

40. Das Verfahren nach Anspruch 38, wobei das biologisch abbaubare Polymer durch Eintauchen in die Zusammensetzung, die das Therapeutikum und den Diblock-oder Triblockträger des Therapeutikums umfasst, beschichtet wird.

41. Das Verfahren nach Anspruch 38, wobei das biologisch abbaubare Polymer durch Besprühen mit der Zusammensetzung, die das Therapeutikum und den Diblock-oder Triblockträger des Therapeutikums umfasst, beschichtet wird.

42. Das Verfahren nach Anspruch 37, das das direkte Anbringen des biologisch abbaubaren Polymers mit der Zusammensetzung, die das Therapeutikum und den Diblock-oder Triblockträger des Therapeutikums umfasst, umfasst.

## Revendications

1. Une enveloppe périvasculaire comportant: (1) un polymère biodégradable, (2) un agent thérapeutique, et (3) un transporteur polymérique de l'agent thérapeutique, dans laquelle le polymère biodégradable est sous la forme de maille, et le transporteur polymérique de l'agent thérapeutique comporte un copolymère di-bloc ou tri-bloc.

2. L'enveloppe périvasculaire selon la revendication 1 dans laquelle le polymère biodégradable est formé à partir d'un ou de plusieurs monomères choisis parmi le groupe constitué du lactide, du glycolide, du ε-caprolactone, du carbonate de tri-méthylène, du 1,4-dioxan-2-one, du 1,5-dioxepan-2-one, du 1,4-dioxepan-2-one, de l'hydroxyvalérate, et de l'hydroxybutyrate.

3. L'enveloppe périvasculaire selon la revendication 1 dans laquelle le polymère biodégradable est un poly(caprolactone), un poly(acide lactique), ou un copolymère de lactide et de ε-caprolactone.

4. L'enveloppe périvasculaire selon la revendication 1 dans laquelle le polymère biodégradable comporte un polyester.

5. L'enveloppe périvasculaire selon la revendication 1 dans laquelle le polymère comporte un poly(lactide-co-glycolide).

6. L'enveloppe périvasculaire selon la revendication 5 dans laquelle le poly(lactide-co-glycolide) est un poly(L-lactide-co-glycolide).

7. L'enveloppe périvasculaire selon la revendication 5 ou la revendication 6 dans laquelle le poly(lactide-co-glycolide) présente un ratio molaire lactide : glycolide est compris entre 3 : 97 et 15 : 85.

8. L'enveloppe périvasculaire selon l'une quelconque des précédentes revendications dans laquelle l'agent thérapeutique est un agent anti-angiogénique.

9. L'enveloppe périvasculaire selon la revendication 8 dans laquelle l'agent anti-angiogénique est du paclitaxel, du fucoidon, de la doxorubicine, ou un analogue ou de dérivé de ceux-ci.

10. L'enveloppe périvasculaire selon la revendication 8 dans laquelle l'agent anti-angiogénique est du paclitaxel.

11. L'enveloppe périvasculaire selon l'une quelconque des revendications de 1 à 7 dans laquelle l'agent thérapeutique est capable d'inhiber au niveau de la cellule musculaire lisse, la migration, la prolifération, la production de matrice, l'inflammation, ou la combinaison de ceux-ci.

12. L'enveloppe périvasculaire selon l'une quelconque des revendications de 1 à 7 dans laquelle l'agent thérapeutique est un agent anti-inflammatoire.

13. L'enveloppe périvasculaire selon l'une quelconque des revendications de 1 à 7 dans laquelle l'agent thérapeutique est un agent antibiotique anti-néoplasique.

14. L'enveloppe périvasculaire selon l'une quelconque des revendications de 1 à 7 dans laquelle l'agent thérapeutique est un oestrogène.

15. L'enveloppe périvasculaire selon l'une quelconque des revendications de 1 à 7 dans laquelle l'agent thérapeutique est un agent antibactérien, un agent antifungique, ou un agent antiviral.

16. L'enveloppe périvasculaire selon l'une quelconque des revendications de 1 à 7 dans laquelle l'agent thérapeutique est un agent antibiotique immunosuppresseur.

17. L'enveloppe périvasculaire selon la revendication 10 comportant de 0,001 mg/cm² à 5 mg/cm² de paclitaxel.

18. L'enveloppe périvasculaire selon la revendication 10 comportant de 0,03 mg/cm² à 0,3 mg/cm² de paclitaxel.

19. L'enveloppe périvasculaire selon l'une quelconque des revendications de 1 à 7 dans laquelle le transporteur polymérique est biodégradable.

20. L'enveloppe périvasculaire selon la revendication 19 dans laquelle le transporteur polymérique biodégradable comporte un copolymère di-bloc ayant un premier bloc et un second bloc, dans lequel le premier boc comprend du méthoxypoly(éthylène glycol) et le second bloc comportant un polyester.

21. L'enveloppe périvasculaire selon la revendication 20 dans laquelle le polyester est un poly(lactide), un poly(glycolide), un poly(caprolactone), un polymère de carbonate de tri-méthylène, un poly(acide hydroxyle), un poly(D,L-lactide-co-glycolide), un poly(L-lactide-co-glycolide), un copolymère d'acide lactique et d'acide glycolique, un copolymère de ε-caprolactone et lactide, un copolymère de glycolide et de ε-caprolactone, ou un copolymère de lactide et 1,4-dioxan-2-one.

22. L'enveloppe périvasculaire selon la revendication 20 dans laquelle le polyester est un polymère ou un copolymère comportant une ou plusieurs des unités résidus des monomères choisis parmi le D-lactide, le L-lactide, le glycolide, le ε-caprolactone, le carbonate de tri méthylène, le 1,4-dioxan-2-one, le 1,5-dioxepan-2-one, l'hydoxyvalérate, et l'hydoxybutyrate.

23. L'enveloppe périvasculaire selon la revendication 20 dans laquelle le polyester est formé à partir d'un ou plusieurs monomère choisi parmi le groupe constitué du lactide, du glycolide, du ε-caprolactone, du carbonate de tri méthylène, du 1,4-dioxan-2-one, du 1,5-dioxepan-2-one, de l'hydoxyvalérate, et de l'hydoxybutyrate.

24. L'enveloppe périvasculaire selon la revendication 21 dans laquelle le poly(lactide) est du poly(D,L-lactide).

25. L'enveloppe périvasculaire selon la revendication 20 dans laquelle le copolymère en di bloc possède un ratio selon le poids de méthoxypoly(éthylène glycol) : polyester compris entre 10 : 90 (± 15%) et 30 : 70 (± 15%).

26. L'enveloppe périvasculaire selon la revendication 20 dans laquelle le copolymère en di bloc possède un ratio selon le poids de méthoxypoly(éthylène glycol) : polyester de 20 : 80 (± 15%).

27. L'enveloppe périvasculaire selon la revendication 24 dans laquelle le ratio selon le poids de méthoxypoly(éthylène glycol) : polyester est de 20 : 80 (±15%).

28. L'enveloppe périvasculaire selon l'une quelconque des revendications 20 - 27 dans laquelle le méthoxypoly(éthylène glycol) possède un poids moléculaire compris entre 200 g/mol (± 15%) et 5 000 g/mol (± 15%).

29. L'enveloppe périvasculaire selon la revendication 28 dans laquelle le méthoxypoly(éthylène glycol) possède un poids moléculaire de 750 g/mol (± 15%).

30. L'enveloppe périvasculaire selon la revendication 19 dans laquelle le transporteur polymérique biodégradable comprend un copolymère tri bloc comportant un structure de A-B-A ou B-A-B, dans laquelle le bloc A comprend un polyoxyalcane, et le bloc B comprends un polyester.

31. L'enveloppe périvasculaire selon la revendication 30 dans laquelle le polyoxyalcane est un poly(éthylène glycol), un poly(oxyde d'éthylène -co- oxyde de propylène), ou un poly(oxyde d'éthylène -co- oxyde de propylène -co- oxyde d'éthylène).

32. L'enveloppe périvasculaire selon la revendication 30 dans laquelle le polyester est un poly(lactide), un poly(glycolide), un poly(caprolactone), un polymère de carbonate de tri méthylène, un poly(acides hydroxyle), un poly(D,L-lactide-co-glycolide), un poly(L-lactide-co-glycolide), un copolymère d'acide lactique et d'acide glycolique, un copolymère de ε-caprolactone et de lactide, un copolymère de glycolide et un ε-caprolactone, ou un copolymère de lactide et un 1,4-dioxan-2-one.

33. L'enveloppe périvasculaire selon la revendication 30 dans laquelle le polyester est un polymère ou un copolymère comportant une ou plusieurs des unités résidus des monomères choisi parmi le D-lactide, le L-lactide, le D,L-lactide, le glycolide, le ε-caprolactone, le carbonate de tri-méthylène, le 1,4-dioxan-2-one, et le 1,5-dioxepan-2-one.

34. L'enveloppe périvasculaire selon la revendication 30 dans laquelle le polyester est formé à partir d'un ou plusieurs monomères choisis parmi le groupe constitué du lactide, du glycolide, du ε-caprolactone, du carbonate de tri méthylène, du 1,4-dioxan-2-one, du 1,5-dioxepan-2-one, du 1,4-dioxepan-2-one, de l'hydroxyvalérate, et de l'hydroxybutyrate.

35. L'enveloppe périvasculaire selon la revendication 1 dans laquelle le polymère biodégradable est du poly(lactide-co-glycolide), l'agent thérapeutique est du paclitaxel, le transporteur polymérique de l'agent thérapeutique est un polymère di bloc ayant un premier bloc comportant du méthoxypoly(éthylène glycol) et un second bloc comportant du poly(D,L-lactide), et l'enveloppe périvasculaire comprenant de 0,03 mg/cm² à 0,3 mg/cm² de paclitaxel.

36. L'enveloppe périvasculaire selon l'une quelconque des revendications de 1 à 35 dans laquelle l'agent thérapeutique et le transporteur polymérique de l'agent thérapeutique forment un revêtement sur le polymère biodégradable.

37. Une méthode pour la production de l'enveloppe périvasculaire selon la revendication 36 comportant l'enrobage ou l'attache directe du polymère biodégradable sous la forme d'un maillage avec une composition comportant un agent thérapeutique et d'un transporteur di bloc ou tri bloc de l'agent thérapeutique.

38. La méthode selon la revendication 37 comportant l'enrobage par le polymère biodégradable avec la composition comportant l'agent thérapeutique et le transporteur di bloc ou tri bloc de l'agent thérapeutique.

39. La méthode selon la revendication 38 dans laquelle le polymère biodégradable est enduit par application de la composition comportant l'agent thérapeutique et le transporteur di bloc ou tri bloc de l'agent thérapeutique.

40. La méthode selon la revendication 38 dans laquelle le polymère biodégradable est enduit par immersion dans la composition comportant l'agent thérapeutique et le transporteur di bloc ou tri bloc de l'agent thérapeutique.

41. La méthode selon la revendication 38 dans laquelle le polymère biodégradable est enduit par spray de la composition comportant l'agent thérapeutique et le transporteur di bloc ou tri bloc de l'agent thérapeutique.

42. La méthode selon la revendication 37 comportant l'attachement directe du polymère biodégradable et de la composition comportant l'agent thérapeutique et le transporteur di bloc ou tri bloc de l'agent thérapeutique.
